(19) Europäisches Patentamt
European Patent Office
Office européen des brevets

(11) Publication number: 0 454 302 A2

# (12) EUROPEAN PATENT APPLICATION

(21) Application number: 91302503.7

(22) Date of filing: 22.03.91

(51) Int. Cl.$^5$: C07K 5/10, C07K 5/12, A61K 37/02

(30) Priority: 30.03.90 JP 83299/90

(43) Date of publication of application:
30.10.91 Bulletin 91/44

(84) Designated Contracting States:
CH DE FR GB IT LI NL

(71) Applicant: BANYU PHARMACEUTICAL CO., LTD.
2-3, Nihonbashi Honcho 2-chome
Chuo-ku Tokyo 103 (JP)

(72) Inventor: Suda, Hiroyuki, c/o Banya Pharmaceutical Co. Ltd.
Exploratory Research Lab, 9-3, Shimomeguro 2-chome
Meguro-ku, Tokyo (JP)
Inventor: Harada, Junko
8-26-403, Uchiya 7-chome
Urawa-shi, Saitama (JP)
Inventor: Tanaka, Seiichi, c/o Banyu Pharmaceutical Co. Ltd
Exploratory Research Lab, 9-3, Shimomeguro 2-chome
Meguro-ku, Tokyo (JP)
Inventor: Koike, Yutaka, c/o Banyu Pharmaceutical Co. Ltd.
Exploratory Research Lab, 9-3, Shimomeguro 2-chome
Meguro-ku, Tokyo (JP)
Inventor: Okura, Akira, c/o Banyu Pharmaceutical Co. Ltd.
Exploratory Research Lab, 9-3, Shimomeguro 2-chome
Meguro-ku, Tokyo (JP)
Inventor: Arakawa, Hiroharu, c/o Banyu Pharmaceutical Co.Ltd
Exploratory Research Lab, 9-3, Shimomeguro 2-chome
Meguro-ku, Tokyo (JP)
Inventor: Okabe, Takayoshi, c/o Banyu Pharmaceutical Co.Ltd
Exploratory Research Lab, 9-3, Shimomeguro 2-chome
Meguro-ku, Tokyo (JP)
Inventor: Okanishi, Masanori c/o Banyu Pharmaceutical Co.Ltd
Exploratory Research Lab, 9-3, Shimomeguro 2-chome
Meguro-ku, Tokyo (JP)

(74) Representative: Davies, Jonathan Mark et al
Reddie & Grose 16 Theobalds Road
London WC1X 8PL (GB)

## (54) Tuftsin derivatives.

(57) Linear or circular tetra peptides which activate immunocompetent cells, i.e., macrophages and polymorphonuclear leukocyte, provide a host-mediated inhibition of the growth of tumors, provide protective effects for infectious diseases such as viral, bacterial and fungal diseases, and exhibit therapeutic effects on autoimmune diseases such as lupus erythematosus, rheumatoid and the like, and therefore, are useful for production of pharmaceutical preparations for the treatment of these diseases.

EP 0 454 302 A2

Jouve, 18, rue Saint-Denis, 75001 PARIS

## BACKGROUND OF THE INVENTION

### 1. Field of the Invention

The present invention relates to peptide derivatives, a process for the production thereof, and a pharmaceutical preparation comprising the derivative.

### 2. Description of the Related Art

Tuftsin (Thr-Lys-Pro-Arg), a naturally occuring or synthetic tetrapeptide having an action to activate immunocompetent cells such as macrophage or polymorphonuclear leucocyte is known (Nishioka et al, Biochimica et Biophysica Acta) 310, 217-229, 1973). As seen from the reference, tuftsin was known, at an early stage of research therein, to be hydrolyzed at its carboxyl terminus by a carboxypeptidase and at its amino terminus by an aminopeptidase.

As a tuftsin derivative resistant to carboxypeptidases, PCT/SU80/0060 discloses a cyclic peptide derivative, theonyl-cyclo(-$N^{\varepsilon}$-lysyl-prolyl-arginyl-). On the other hand, EP 296059 discloses a tuftsin analog synthesized on the basis of research relating to an aminopeptidase inhibitor (Nishizawa et al, J. Medicinal Chemistry, 20, 510-515, 1977).

## SUMMARY OF THE INVENTION

The present invention provides new peptide derivatives which effectively activate the phagocytic action of macrophages and polymorphonuclear leukocytes, and exhibit a superior therapeutic action against the immunodeficiency syndrome.

More particularly, the present invention provide a peptide derivative represented by the following formula (I):

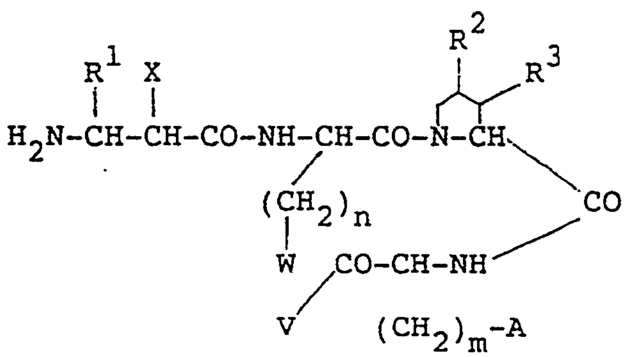

(I)

wherein: V represents a hydroxyl group;

W represents a hydrogen atom, lower alkyl, cycloalkyl having three to seven carbon atoms, phenyl, amino, mono- or di-lower alkylamino, amidino, or guanidino group; or

W and V together form a group of the formula:

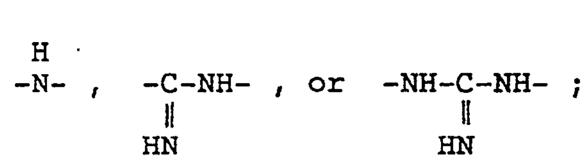

X represents a hydrogen atom, or a hydroxy or mercapto group;

$R^1$ represents a hydrogen atom, or a phenyl, p-hydroxyphenyl or lower alkyl group, which lower alkyl group is optionally substituted with one to three of the same or different substituents selected from the group consisting of amino, hydroxy, lower alkoxy, mercapto, lower alkylthio, lower alkylsulfinyl, lower alkylsulfonyl, carboxyl, aryl, heteroaryl, cycloalkyl having three to seven carbon atoms and of bicycloalkyl having eight to ten carbon atoms, wherein the aryl, heteroaryl, cycloalkyl or bicycloalkyl group is optionally substituted with hydroxy, lower alkoxy, lower alkyl, nitro, amino, mono- or di-lower alkylamino, mercapto, lower alkylthio, lower alkylfulfinyl or with lower

alkylsulfonyl;

$R^2$ and $R^3$ are the same and represent a hydrogen atom or a lower alkyl group; or one of $R^2$ and $R^3$ represents a hydrogen atom, and the other represents a hydroxy, lower alkoxy or lower alkyl group; or both $R^2$ and $R^3$ together form a single bond;

A represents an amino, mono- or di-lower alkylamino, amidino, or guanidino group; and

m and n independently represent an integer of 0 to 4;

and pharmaceutically acceptable salts thereof.

The present invention also provides a peptide derivative represented by the formula (II):

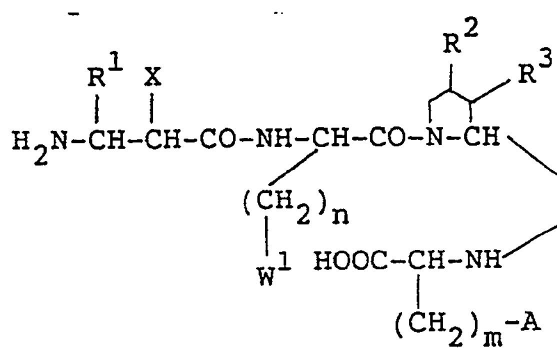

(II)

wherein: X represents a hydrogen atom, or a hydroxy or mercapto group;

$W^1$ represents a hydrogen atom, lower alkyl, cycloalkyl having three to seven carbon atoms, phenyl, amino, mono- or di-lower alkylamino, amidino, or guanidino group;

$R^1$ represents a hydrogen atom, or a phenyl, p-hydroxyphenyl or lower alkyl group, which lower alkyl group is optionally substituted with one to three of the same or different substituents selected from the group consisting of amino, hydroxy, lower alkoxy, mercapto, lower alkylthio, lower alkylsulfinyl, lower alkylsufonyl, carboxyl, aryl, heteroaryl, cycloalkyl having three to seven carbon atoms, and of bicycloalkyl having eight to ten carbon atoms, wherein the aryl, heteroaryl, cycloalkyl or bicycloalkyl group is optionally substituted with a hydroxy, lower alkyloxy, lower alkyl, nitro, amino, mono- or di-lower alkylamino, mercapto, lower alkylthio, lower alkylfulfinyl or with lower alkylsulfonyl;

$R^2$ and $R^3$ are the same and represent a hydrogen atom or a lower alkyl group; or one of $R^2$ and $R^3$ represents a hydrogen atom, and the other represents a hydroxy, lower alkoxy or lower alkyl group; or both $R^2$ and $R^3$ together form a single bond;

A represents an amino, mono- or di-lower alkylamino, amidino, or guanidino group; and

m and n independently represent an integer of 0 to 4; and

pharmaceutically acceptable salts thereof.

The present invention moreover provides a peptide derivative represented by the formula (III):

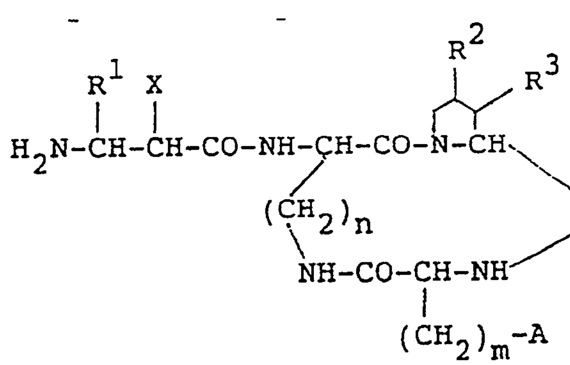

(III)

wherein: X represents a hydrogen atom; or a hydroxy or mercapto group;

$R^1$ represents a hydrogen atom, or a phenyl, p-hydroxyphenyl or lower alkyl group, which lower alkyl group is optionally substituted with one to three of the same or different substituents selected from the group consisting of amino, hydroxy, lower alkoxy, mercapto, lower alkylthio, lower alkylsulfinyl, lower alkylsulfonyl, carboxyl, aryl, heteroaryl, cycloalkyl having three to seven carbon atoms and of bicycloalkyl having eight to ten carbon atoms, wherein the aryl, heteroaryl, cycloalkyl or bicycloalkyl group is optionally substituted with a hydroxy, lower alkyloxy, lower alkyl, nitro, amino, mono- or di-lower alkylamino, mercapto, lower alkylthio, lower alkylsulfinyl

or with lower alkylsulfonyl;

$R^2$ and $R^3$ are the same and represent a hydrogen atom or a lower alkyl group; or one of $R^2$ and $R^3$ represents a hydrogen atom, and the other represents a hydroxy, lower alkoxy or lower alkyl group; or both $R^2$ and $R^3$ together form a single bond;

A represents an amino, mono- or di-lower alkylamino, amidino, or guanidino group; and

m and n independently represent an integer of 0 to 4; and

pharmaceutically acceptable salts thereof.

A preferred peptide derivative of the present invention is selected from the group consisting of:

AHPA-cyclo(-$N^{\varepsilon}$-(S)-lysyl-(S)-prolyl-(S)-arginyl-);
AHCA-cyclo(-$N^{\varepsilon}$-(S)-lysyl-(S)-prolyl-(S)-arginyl-);
3-amino-2-hydroxy-4-(4-hydroxyphenyl) butanoyl-cyclo(-$N^{\varepsilon}$-(S)-lysyl-(S)-prolyl-(S)-arginyl-);
3-amino-2-hydroxybutanoyl-cyclo(-$N^{\varepsilon}$-(S)-lysyl-(S)-prolyl-(S)-arginyl-);
AHPA-cyclo(-$N^{\delta}$-(S)-ornithyl-(S)-prolyl-(S)-arginyl-);
3-amino-2-hydroxy-4-(4-methoxyphenyl) butanoyl-cyclo(-$N^{\varepsilon}$-(S)-lysyl-(S)-prolyl-(S)-arginyl-);
3-amino-2,4-dihydroxypentanoyl-cyclo(-$N^{\varepsilon}$-(S)-lysyl-(S)-prolyl-(S)-arginyl-);
$N^{\alpha}$-AHCA-(S)-lysyl-(S)-prolyl-(S)-arginine;
$N^{\alpha}$-(3-amino-2-hydroxy-4-(4-methoxyphenyl butanoyl)-(S)-lysyl-(S)-prolyl-(S)-arginine;
$N^{\alpha}$-(3-amino-2-hydroxybutanoyl)-(S)-lysyl-(S)-prolyl-(S)-arginine;
$N^{\alpha}$-AHCA-(S)-leucyl-(S)-prolyl-(S)-arginine;
$N^{\alpha}$-AHCA-(S)-cyclohexylalanyl-(S)-prolyl-(S)-arginine;
and a pharmaceutically acceptable salt thereof.

Another preferred peptide derivative is selected from the group consisting of:

(2S, 3R)-AHPA-cyclo(-$N^{\varepsilon}$-(S)-lysyl-(S)-prolyl-(S)-arginyl-);
(2S, 3R)-AHCA-cyclo(-$N^{\varepsilon}$-(S)-lysyl-(S)-prolyl-(S)-arginyl-);
(2S, 3R)-3-amino-2-hydroxy-4-(4-hydroxyphenyl) butanoyl-cyclo(-$N^{\varepsilon}$-(S)-lysyl-(S)-prolyl-(S)-arginyl-);
(2S, 3R)-3-amino-2-hydroxybutanoyl-cyclo(-$N^{\varepsilon}$-(S)-lysyl-(S)-prolyl-(S)-arginyl-);
(2S, 3R)-AHPA-cyclo(-$N^{\delta}$-(S)-ornithyl-(S)-prolyl-(S)-arginyl-);
(2S, 3R)-3-amino-2-hydroxy-4-(4-methoxyphenyl) butanoyl-cyclo(-$N^{\varepsilon}$-(S)-lysyl-(S)-prolyl-(S)-arginyl-);
(2S, 3R, 4S)-3-amino-2,4-dihydroxypentanoyl-cyclo(-$N^{\varepsilon}$-(S)-lysyl-(S)-prolyl-(S)-arginyl-);
$N^{\alpha}$-((2S, 3R)-AHCA)-(S)-lysyl-(S)-prolyl-(S)-arginine;
$N^{\alpha}$-((2S, 3R)-3-amino-2-hydroxy-4-(4-methoxyphenyl) butanoyl)-(S)-lysyl-(S)-prolyl-(S)-arginine;
$N^{\alpha}$-((2S, 3R)-3-amino-2-hydroxybutanoyl)-(S)-lysyl-(S)-prolyl-(S)-arginine;
$N^{\alpha}$-((2S, 3R)-AHCA)-(S)-leucyl-(S)-prolyl-(S)-arginine; and
$N^{\alpha}$-((2S, 3R)-AHCA)-(S)-cyclohexylalanyl-(S)-prolyl-(S)-arginine;
and pharmaceutically active salt thereof.

The present invention also provides pharmaceutical preparations comprising the above-mentioned peptide derivative or a pharmaceutically acceptable salt thereof, together with a conventional carrier.

The present invention still further provides a process for a peptide derivative of the formula (I), comprising the steps of;

(1) reacting a compound represented by the following formula (IV):

$$\begin{array}{c} \quad R^1 \; X \\ H_2N\text{-}\underset{}{CH}\text{-}CH\text{-}COOH \end{array} \qquad (IV)$$

wherein $R^1$ and X have the same meanings as defined above, or a corresponding compound wherein an amino group, a functional group if present in the $R^1$, and/or a mercapto or hydroxy group if X is a mercapto or hydroxy group, are protected, with a compound represented by the following formula (V):

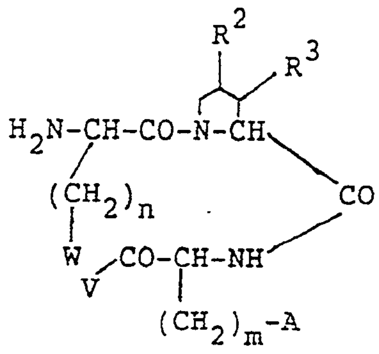

(V)

wherein V, W, $R^2$, $R^3$, A, m and n have the same meanings as defined above, or with a corresponding compound B) - (V) wherein one or more functional groups in the formula (V) are protected, in the presence of a condensation agent, and if necessary, in the presence of a base; and optionally,

(2) eliminating the protective groups.

The term "lower" as used herein refers to a group or compound having up to 6, preferably up to 4 carbon atoms.

Therefore, a lower alkyl group is an linear or branched alkyl group having 1 to 6 carbon atoms, such as methyl, ethyl, propyl, isopropyl, butyl, isobutyl, sec-butyl, tert-butyl, pentyl, isopentyl, neopentyl or hexyl group, or the like.

The lower alkoxy group is a linear or branched alkoxy group having 1 to 6 carbon atoms, such as methoxy, ethoxy, propoxy , sec-propoxy, butoxy, pentyloxy or hexyloxy group, or the like.

The lower alkylthio group is a linear or branched alkylthio group having 1 to 6 carbon atoms, such as methylthio, ethylthio, propylthio, buthylthio, pentylthio or hexylthio group, or the like.

The lower alkylsulfinyl group is a linear or branched alkylsulfinyl group having 1 to 6 carbon atoms, such as methylsulfinyl, ethylsulfinyl, propylsulfinyl, isopropylsulfinyl, butylsulfinyl, sec-butylsulfinyl, pentylsulfinyl or hexylsulfinyl group, or the like.

The lower alkylsulfonyl is a linear or branched alkylsulfonyl group having 1 to 6 carbon atoms; such as methylsulfonyl, ethylsulfonyl, propylsulfonyl, isopropylsulfonyl butylsulfonyl, sec-butylsulfonyl, pentylsulfonyl or hexylsulfonyl group, or the like.

The aryl group is an aromatic hydrocarbon group having 6 to 10 carbon atoms, such as phenyl, naphthyl or tetrahydronaphthyl group, or the like.

The heteroaryl group is a heteroaryl group containing 1 or 2 hetero atoms such as oxygen, sulfur and/or nitrogen atoms, for example, thienyl, pyridyl, indolyl, thiazolyl or oxazolgl group.

A cycloalkyl group having 3 to 7 carbon atoms is a cyclic hydrocarbon group such as cyclopropyl, cyclobutyl, cyclopentyl, cyclohexyl or cycloheptyl group, or the like.

A bicycloalkyl group having 8 to 10 carbon atoms is a condensed cyclic hydrocarbon group, such as bicyclo [4. 4. 0] decyl or bicyclo [4. 3. 0] nonyl group, or the like.

The abbreviations as used herein have the following meanings:

| Abbreviation | Meaning |
|---|---|
| AHPA | 3-amino-2-hydroxy-4-phenylbutanoyl group |
| AHCA | 3-amino-2-hydroxy-4-cyclohexylbutanoyl group |
| DCC | N,N'-Dicyclohexylcarbodiimide |
| DMF | N,N-Dimethylformamide |
| EDC | 1-Ethyl-3-(3-dimethylaminopropyl) carbodiimide |
| HOBt | 1-Hydroxybenzotriazole |
| THF | Tetrahydrofuran |

Next, to further explain the present compound in detail, the symbols used in the formula (I) are clearly defined.

Namely, $R^1$ represents a hydrogen atom, or phenyl, p-hydroxyphenyl or lower alkyl group, wherein the lower alkyl group is optionally substituted with 1 to 3 same or different substituents selected from the group consisting of amino group, hydroxy group, lower alkoxy group, mercapto group, lower alkylthio group, lower alkylsulfinyl group, lower alkylsulfonyl group, carboxyl group, aryl group, heteroaryl group, cycloalkyl group having 3 to 7 carbon atoms and bicycloalkyl group having 8 to 10 carbon atoms, wherein the aryl, heteroaryl, cycloalkyl and/or bicycloalkyl groups are optionally in turn substituted with a hydroxy, lower alkoxy, lower alkyl, nitro, amino, mono- or di-lower alkylamino, mercapto, lower alkylthio, lower alkylsulfinyl or lower alkylsulfonyl group.

As examples of $R^1$, there are mentioned a hydrogen atom; a lower alkyl group, such as a methyl, ethyl, propyl, isopropyl, sec-butyl, butyl, pentyl, isopentyl, hexyl or isohexyl group; an alkyl group having an amino group, such as an aminomethyl, 2-aminoethyl, 1-aminopropyl, 1-aminopentyl or 3-aminopentyl group; a lower alkyl group having a hydroxyl group, such as hydroxymethyl, 2-hydroxyethyl, 1-hydroxypropyl or 2-hydroxypentyl group; a lower alkyl group having a lower alkoxy, such as 1-methoxyethyl, 2-methoxyethyl, 3-methoxypropyl, 2-methoxypropyl, 4-methoxybutyl, 4-methoxypentyl, 5-methoxypentyl, 5-methoxyhexyl, 4-ethoxypentyl, 5-ethoxypentyl, 3-ethoxyhexyl, 4-ethoxyhexyl, 5-ethoxyhexyl, 4-propoxypentyl, 5-propoxypentyl or 2-hexyloxyethyl group; a lower alkyl having a mercapto group, such as 1-mercaptoethyl, 2-mercaptoethyl, 3-mercaptopropyl, 4-mercaptobutyl, 3-mercaptopentyl or 3-mercaptohexyl group; a lower alkyl group having a lower alkylthio group, such as 1-methylthioethyl, 2-methylthioethyl, 3-methylthiopropyl, 4-methylthiobutyl, 5-methylthiopentyl 4-ethylthiopentyl, 5-ethylthiopentyl, 3-ethylthiohexyl, 4-propylthiopentyl or 2-hexylthioethyl group; a lower alkyl group having a lower alkylsulfinyl group, such as 1-methylsulfinylethyl, 2-methylsulfinylethyl, 3-methylsulfinylpropyl, 4-methylsulfinylbutyl, 5-methylsulfinylpentyl, 4-ethylsulfinylpentyl, 5-ethylsulfinylpentyl or 3-ethylsulfinylhexyl group; a lower alkyl group having a lower alkylsulfonyl group, such as 2-methylsulfonylmethyl, 2-methylsulfonylethyl, 3-methylsulfonylpropyl, 3-methylsulfonylbutyl, 5-methylsulfonylpentyl, 4-ethylsulfonylpentyl, 5-ethylsulfonylpentyl, 4-ethylsulfonylhexyl or 4-propylsulfonylpentyl group; a lower alkyl group having a carboxyl group, such as carboxymethyl, 2-carboxyethyl, 1-carboxyethyl, 3-carboxypropyl, 3-carboxybutyl, 5-carboxypentyl or 2-carboxyhexyl group; a lower alkyl group having an aryl group, such as benzyl, α-methylbenzyl, phenethyl, 1-phenylpropyl, 2-phenylpropyl, 1-phenylbutyl, 1-phenylpentyl, 2-phenylhexyl, 1-naphtylmethyl, 2-(1-naphtyl)ethyl, 5-(1-naphtyl)pentyl, 6-(2-naphtyl)hexyl, [1-(1,2,3,4-tetrahydronaphtyl]methyl or 2-[1-(1,2,3,4-tetrahydronaphtyl)]ethyl group; a lower alkyl group having a heteroaryl group, such as 2-thienylmethyl, 3-thienylmethyl, 1-(2-thienyl)ethyl, 2-pyridylmethyl, 3-indolylmethyl, 4-thiazolylmethyl or 2-oxazolydylmethyl group; a lower alkyl group substituted with a cycloalkyl group having 3 to 7 carbon atoms, such as cyclopropylmethyl, cyclobutylmethyl, cyclopentylmethyl, cyclohexylmethyl, 1-cyclohexylethyl, 2-cyclohexylethyl, 3-cyclohexylpropyl, 3-cyclohexylpentyl, 1-cyclohexylbutyl or cycloheptylmethyl group; or a lower alkyl group substituted with a bicycloalkyl group having 8 to 10 carbon atoms, such as bicyclo[4.4.0] decyl or bicyclo [4.3.0] nonyl group; and moreover, in the lower alkyl group substituted with an aryl group, with a heteroaryl group, with a cycloalkyl having 3 to 7 carbon atoms or with a bicycloalkyl group having 8 to 10 carbon atoms, a hydrogen atom on the ring moiety can be substituted with a hydroxyl, lower alkoxy, lower alkyl, nitro,

amino, mono- or di-lower alkyl amino, mercapto, lower alkylthio, lower alkylsulfinyl or lower alkylsulfonyl group. Moreover, $R^1$ can be a lower alkyl group substituted with 2 or 3 of the same or different substituents selected from the group consisting of an amino group, hydroxy group, lower alkoxy group, mercapto group, lower alkylthio group, lower alkylsulfinyl group, lower alkylsulfonyl group, carboxyl group, aryl group, heteroaryl group, cycloalkyl group having 3 to 7 carbon atoms and bicycloalkyl group having 8 to 10 carbon atoms.

$R^2$ and $R^3$ are the same and represent a hydrogen atom or lower alkyl group; or one thereof represents a hydrogen atom and the other represents a hydroxy, lower alkoxy or lower alkyl group; or $R^2$ and $R^3$ together form a single bond.

As examples of $R^2$ and $R^3$ there are mentioned a hydrogen atom; a hydroxy group; a lower alkoxy group, such as a methoxy, ethoxy, propoxy, butoxy, tert-butoxy, pentyloxy, isopentyloxy, hexyloxy or isohexyloxy group; or a lower alkyl group, such as a methyl, ethyl, propyl, isopropyl, butyl, sec-butyl, tert-butyl, isopentyl, hexyl or isohexyl group. Where $R^2$ and $R^3$ together form a single bond, the amino acid carrying $R^2$ and $R^3$ is 3,4-dehydroproline.

Further, m and n are independently an integer of 0 to 4, and A and W can independently represent an amino, mono- or di-lower alkyl amino, amidino or guanidino group, and therefore, when m or n is 3, the amino acid carrying the A or W may be, for example, ornithine, $N^{\delta}$-methylornithine, $N^{\delta}$-dimethylornithine, 5-amidino-2-aminopentanoic acid, arginine or the like, and when m or n is 4, the amino acid carrying the A or W may be, for example, lysine, $N^{\varepsilon}$-methyllysine, $N^{\varepsilon}$-dimethyllysine, 6-amidino-2-aminohexanoic acid, 2-amino-6-guanidinohexanoic acid, or the like. Moreover, W represents a hydrogen atom, lower alkyl, cycloalkyl having three to seven carbon atoms or phenyl group, and therefore the amino acid carrying W may be glycine, alanine, valine, leucine, norleucine, cyclohexylalamine, phenylalamine, or the like.

Next a process for the production of the present invention is described.

A compound of the present invention can be produced by reacting a compound represented by the following formula (IV):

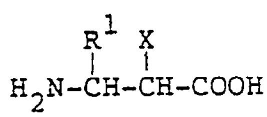

(IV)

wherein $R^1$ and X have the same meaning as described above, or a corresponding compound B-(IV) wherein the amino group in the formula (IV), a functional group if present in $R^1$, and/or mercapto or hydroxy group if X is a mercapto or hydroxy group are protected, with a compound represented by the formula (V):

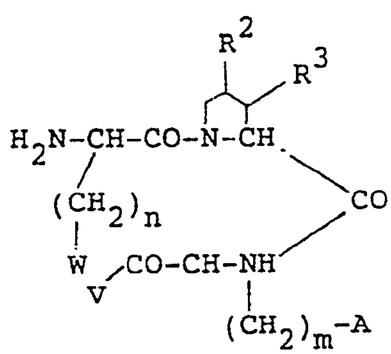

(V)

wherein V, W, A, $R^2$, $R^3$, m and n have the same meanings as defined above, or a corresponding compound B-(V) wherein one or more than one functional group in the formula (V) is protected, in the presence of a condensation agent, and if necessary, in the presence of a base; and then, if necessary, by eliminating the protecting groups.

The reaction of the compound (IV) or B-(IV) and the peptide compound (V) or B-(V) is a condensation reaction between a carboxyl group and an amino group as well known in the field of peptide chemistry. Accordingly, the reaction of the compound (IV) or B-(IV) and the compound (V) or B-(V) can be carried out according to a conventional procedure in the field of peptide chemistry; for example, in a solvent not providing adverse effects on the reaction in the presence of a condensation agent, and if necessary, in the presence of a condensation aid such as a suitable base and/or N-hydroxysuccinimide or 1-hydroxybenzotriazole.

As a condensation agent, for example, carbodiimide such as dicyclohexylcarbodiimide (DCC), 1-ethyl-3-(3-dimethylaminopropyl)carbodiimide (EDC) is used.

As an organic solvent used in the condensation reaction, an ether such as ethyl ether, tetrahydrofuran dioxane or the like, an ester such as ethyl acetate or methyl acetate, ketone such as acetone, methyl ethyl ketone or the like, a chlorinated hydrocarbon such as methylenechloride, chloroform or the like, an aprotic polar solvent such as dimethylformamide, dimethylacetamide, acetonitrile or the like, or a mixture thereof, is used.

As a suitable base, for example, an inorganic base such as sodium bicarbonate, magnesium oxide or the like, or an organic base such as triethylamine, N-methylmorpholine or the like, is used.

Compound (IV) or B-(IV) is used usually in an equivalent amount or if necessary, 0.7 to 2.0 mole amount relative to the amount of compound (V) or B-(V); a base is used in a catalytic amount to 2 mole amount, and preferably, an equivalent amount relating to the amount of compound (V) or B-(V). The condensation agent is used usually in a small excess amount.

The reaction is carried out usually at an temperature between -50°C and 50°C, or if necessary at a temperature higher or lower than said range, usually for 30 minutes to days, or if necessary, for a term lower or shorten than said range.

After a condensation reaction, or after an elimination of protecting groups, depending on the reaction condition, a desired product is purified according to a conventional process in the field of organic chemistry, for example, by solvent extraction, recrystallization, or chromatography, or by the method, using an ion exchange resin.

The protection of a functional group in a starting compound is carried out by using a conventional protecting group commonly used in peptide chemistry. For example, a tert-butoxy carbonyl or benzyloxycarbonyl group is preferably used as a protecting group for an amino group; a mesitylsulfonyl group is preferably used as a protecting group for an amidino or guanidino group; a tetrahydropyranyl, tert-butoxy, benzyl or alkanoyl, group is preferably used as a protecting group for a hydroxy group; and a carboxyl group is preferably protected in the form of an alkyl ester or benzyl ester.

Deprotection can be carried out according to a procedure conventionally used in peptide chemistry, for example, by catalytic hydrogenation using palladium-carbon, palladium black or the like, or acidic hydrolysis using hydrogen bromide in acetic acid, trifluoroacetic acid, p-toluenesulfonic acid in an organic solvent such as dioxane, tetrahydrofuran or the like, or hydrogen chloride.

A condensation of compound (IV) or B-(IV) with compound (V) or B-(V) also can be carried out by a mixed acid anhydride method, wherein first the compound (IV) or B-(IV) is reacted with a chloroformate such as ethylchloroformate or isobutylchloroformate in a suitable solvent in the presence of a base, to form a mixed acid anhydride, which is then condensed with the compound (V) or B-(V).

The solvent and base used in the above-mentioned condensation reaction may be same as those used in the afore-mentioned condensation process using a carbodiimide-type condensation agent such as DCC or EDC.

In the reaction, compound (V) or B-(V) is used usually in a small excess amount relative to the amount of compound (IV) or B(IV), or if necessary, in an amount more or less than said amount. An amount of base used is usually 1.0 or 2.0 mole equivalent relative to the amount of compound (V) or B-(V).

After the condensation reaction, or if necessary, after the elimination of protective groups, a desired product can be purified according to a procedure known in the field of organic chemistry, for example, by solvent extraction, recrystallization, or chromatography, or by the method, using an ion exchange resin, or the like.

The kind of protecting agent and the deprotection can be the same as those used for the afore-mentioned condensation process using DCC or EDC as a condensation agent.

A compound thus synthesized may be obtained as a single steric isomer or as a mixture of steric isomers; and the present invention includes both the single steric isomer and the mixture of steric isomers. A mixture of steric isomers can be separated according to a conventional procedure.

Moreover, the present invention includes pharmaceutically acceptable salts of the compound (I). As the salts, there are mentioned salt with an inorganic acid such as hydrochloric acid, hydrogen bromide, sulfuric acid, phosphoric acid or the like; salt with an organic acid such as acetic acid, organic sulfuric acid, tartaric acid, succinic acid, fumaric acid, citric acid, maleic acid, aspartic acid, glutamic acid, benzoic acid, glycolic acid or the like; and salt with an acidic amino acid.

The starting compounds (V) and B-(V) are a linear peptide or cyclic tri-peptide.

The cyclic peptide can be easily produced by cyclizing a linear tripeptide represented by the following formula (VI):

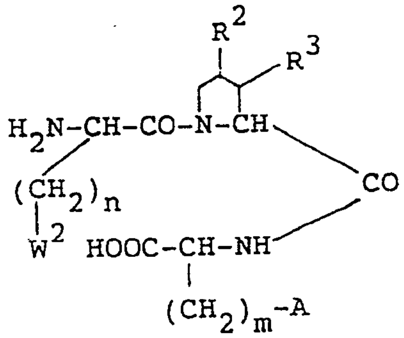

(VI)

wherein $W^2$ represents an amino, mono- or di-lower alkylamino, amidino or guanidino group; and A, $R^2$, $R^3$ m and n have the same meanings as defined above, or a compound B-(VI) wherein the α-amino group, amino, mono-or di-lower alkylamino, amidino or guanidino group for A, and/or hydroxyl group when $R^2$ or $R^3$ is a hydroxyl group are protected.

The linear tripeptides (VI) and B-(VI) can be easily produced according to a conventional procedure well known in the field peptide synthesis. Namely, these compounds are obtained by optionally protecting component amino acids with suitable protecting agents, and subsequently condensing the component amino acids by a liquid phase or solid phase method.

The condensation can be carried out, for example, by the carbodiimide method, mixed acid anhydride method, azide method or active ester method.

The condensation, deprotection and purification of a desired product can be carried out according to the same procedures as described for the compound (I) obtained from the compound (IV) or B-(IV) and the compound (V) or B-(V).

Next, a process for the production of the compound (V) and B-(V) is explained in detail.

The compounds (V) and B-(V) are a cyclic or linear tri-peptide, and contain 3 amide bonds in the case of a cyclic peptide or 2 amide bonds in the case of a linear peptide. The order or the condensation when forming these amide bonds is optional and is not critical in the synthesis of the compounds (V) and B-(V), and thus in the synthesis of the compound (I) and B-(I) incorporating protecting groups.

As an example, the compound (V) and B-(V) can be produced as follows.

First, a compound represented by the following formula (VII):

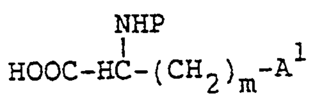

(VII)

wherein $A^1$ represents an optionally protected amino, mono- or di-lower alkylamino, amidino or guanidino group, P represents an amino protecting group, and m has the same meaning as defined above, wherein the condition for the elimination of P does not adversely affect $A^1$, is reacted for example, with benzylbromide in the presence of a suitable base to protect the carboxyl group to obtain a compound represented by the following formula (VIII):

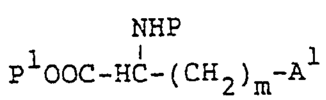

(VIII)

wherein $P^1$ represents a carboxyl protecting group, and $A^1$, P and m have the same meaning as defined above, wherein the condition for eliminating P does not adversely affect $A^1$ and $P^1$.

For example, a protecting group in $A^1$ is preferably mesitylsulfonyl group, and the protective group P is preferably tert-butoxycarbonyl group.

Next, the protecting group P in the compound (VIII) is eliminated, for example, by an acid catalyst such as p-toluenesulfonic acid, if the protecting group is tert-butoxycarbonyl, to obtain a compound represented by the following formula (IX):

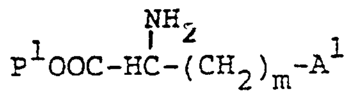

(IX)

wherein $A^1$ and $P^1$ have the same meanings as defined above.

Next, the compound (IX) thus obtained is condensed with a compound represented by the following formula (X):

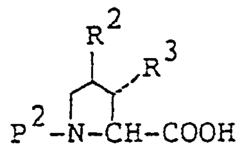

(X)

wherein $P^2$ represents an imino protecting group, and $R^2$ and $R^3$ have the same meaning as defined above, wherein the condition for eliminating $P^2$ does not adversely affect $A^1$ and $P^1$, or with a corresponding compound wherein, if $R^2$ or $R^3$ is a hydroxyl group, this hydroxyl group is protected, to obtain a compound represented by the formula (XI):

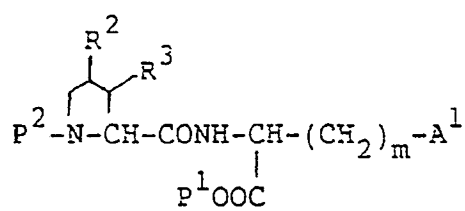

(XI)

wherein $A^1$, $P^1$, $P^2$, $R^2$, $R^3$ and m have the same meanings as defined above, or a corresponding compound wherein, if $R^2$ or $R^3$ is a hydroxyl group, this hydroxyl group is protected.

The condensation condition used in the above reaction may be the same as that used for the condensation of the compound (IV) or B-(IV) with the compound (V) or B-(V) as described above.

The protecting group $P^2$ for the imino group on the pyrrolidine ring is preferably a tert-butoxycarbonyl group.

Next, the imino protecting group $P^2$ in the compound (XI) is elimitated, for example, in the presence of an acid catalyst such as p-toluenesulfonic acid, if $P^2$ is a tert-butoxycarbony group, to obtain a corresponding compound (XII) wherein the imino group is deprotected, and the compound (XII) is then condensed with a compound represented by the folowing formula (XIII):

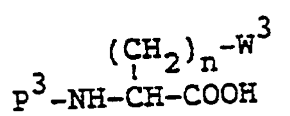

(XIII)

wherein $W^3$ represents a hydrogen atom, lower alkyl, cycloalkyl having three to seven carbon atoms, phenyl, optionally protected imino, mono- or di-lower alkylamino, amidino or guanidino group, and $P^3$ represents an amino protecting group, wherein the condition for eliminating a protecting group for $W^3$ does not adversely affect $A^1$ and $P^3$, and n has the same meaning as described above, to obtain a protected linear tripeptide represented by the following formula (XIV):

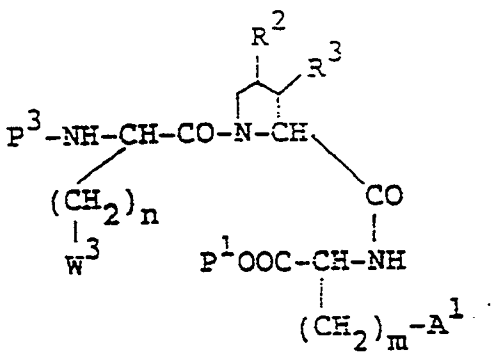

(XIV)

wherein $W^3$, $A^1$, $R^2$ $R^3$, $P^1$, $P^3$, m and n have the same meanings as defined above.

In the formula (XIII) and (XIV), the protecting group for $W^3$ is preferably a benzyloxycarbonyl group, and the protecting group $P^3$ is preferably a tert-butoxycarbonyl group.

After eliminating $P^3$ in the compound (XIV), a resulting compound is condensed with the compound (IV) or B-(IV), and if necessary, existing protecting groups are eliminated to obtain a linear peptide derivative of the present invention.

On the other hand, when the protecting group for $W^3$ and the protecting group $P^1$ in the formula (XIV) are eliminated, a compound represented by the following formula (XV):

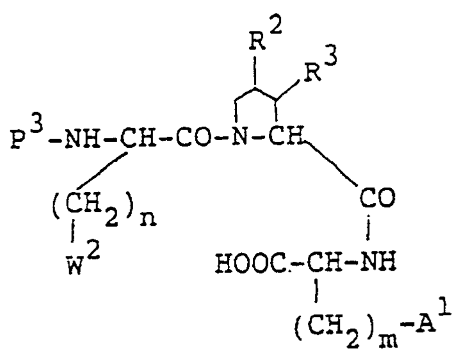

(XV)

wherein $W^2$, $A^1$, $R^2$, $R^3$, $P^3$, m and n have the same meaning as defined above, is obtained. Next, the compound (XV) is intramolecularly condensed by using a suitable condensation agent such as EDC, to obtain a protected cyclic tri-peptide derivative represented by the following formula (XVI):

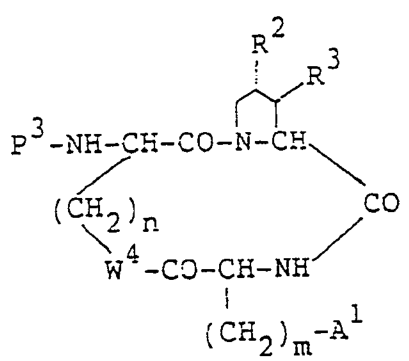

(XVI)

wherein $A^1$, $R^2$, $R^3$, $P^3$, m and n have the same meaning as defined above, and $W^4$ represents

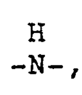 , 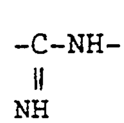 , or 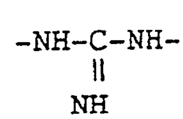 .

Where the protecting group for $W^3$ of the compound (XIV) is benzyloxycarbonyl group, this can be eliminated by a hydrogenation, for example, a palladium black as a catalyst. Where the carboxyl protecting group $P^1$ is a benzyl group, this benzyl group can be eliminated simultaneously with the elimination of the benzyloxycarbonyl group which is a protecting group for $W^3$. To produce the cyclic tri-peptide, this condition can be advantageously used.

A tert-butoxycarbonyl group can be preferably used for $P^3$, and this protecting group can be easily deprotected by trifluoroacetic acid.

The starting materials of the present process may be known amino acids produced by fermentation or chemical synthesis, or natural amino acids or amino acids prepared by hydrolysis of a protein. Moreover, as starting materials, a compound prepared from an amino acid by changing the number of carbon atoms using a chemical means, or side chain-modified amino acids, can be used.

Particularly, among compounds represented by the general formula (IV), compounds wherein X is a hydroxyl group and the corresponding compounds wherein functional groups are protected can be produced, for example, by protecting the amino group of a starting material, α-amino acid, condensing the protected amino acid with pyrazole or 3,5-dimethylpyrazole using a condensation agent, dicyclohexylcarbodiimide (DCC), reducing the condensation product with lithium aluminum hydride ($LiAlH_4$), converting the resulting aldehyde of N-protected amino acid to the corresponding cyanohydrine derivative, and hydrolyzing the product, as known from Suda et al., J. Antibiotics, 29, 600-610, 1976; and Nishizawa et al., J. Medicinal Chemistry, 20, 510-545 (1977).

Alternatively, a compound of the formula (IV) can be stereo-selectively produced according to the following reaction scheme:

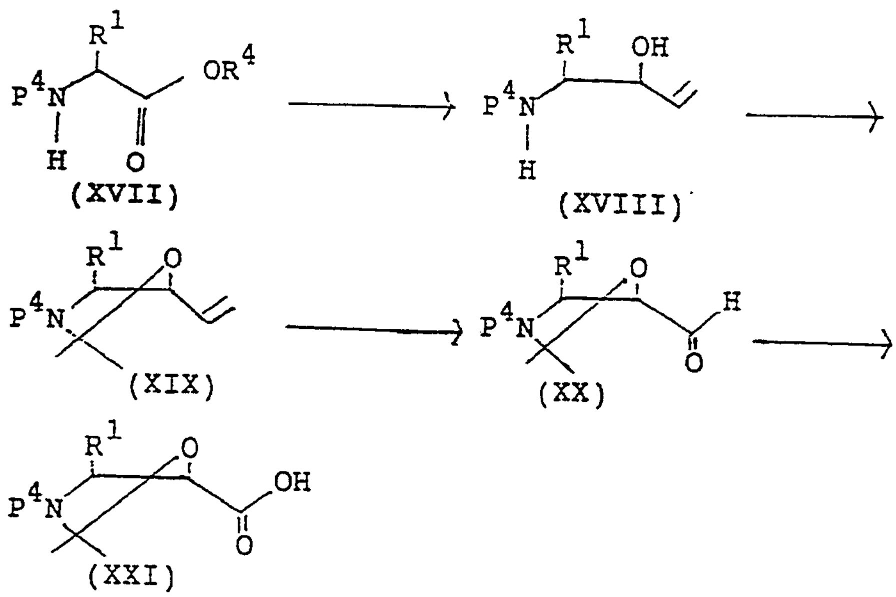
(XVII) (XVIII) (XIX) (XX) (XXI)

wherein $R^4$ represents a lower alkyl group, $P^4$ represents an amino protecting group, and $R^1$ has the same meaning as defined above.

More specifically, a lower alkyl ester of an amino-protected D- or L- amino acid represented by the formula (XVII) is reduced to a corresponding aldehyde using as a reducing agent, such as diisobutyl aluminium hydride, and the aldehyde is reacted with vinyl magnesium bromide to obtain a corresponding 1-en-3-ol compound represented by the formula (XVIII). Next, the 1-en-3-ol compound is converted to a compound represented by the general formula (XIX) by forming an oxazolidine ring using 2,2-dimethoxypropane, and the compound (XIX) is reacted with osmium tetraoxide to obtain a 5-formyl-oxazoline derivative represented by the general formula (XX), which is then oxidized with Jones reagent to obtain a compound represented by the general formula (IV). If necessary, a protected compound of the formula (IV) can be deprotected to obtain a corresponding compound wherein X is a hydroxy group.

Moreover, a compound represented by the general formula (IV) or a corresponding protected compound B-(IV) wherein X is a mercapto group can be produced by a known process, for example, by converting an α-amino acid optionally protected at the amino group to a corresponding 1-diazo-2-one derivative using isobutylchloroformate, N-methylmorpholine and diazomethane, converting the product to a corresponding methyl

ester of the compound wherein X is a hydrogen atom (this compound can be a starting material used to prepare the present compound (I) wherein X is a hydrogen atom) using silver benzoate and methanol, reacting the product with liyhium diisopropylamide and then with 4-methoxybenzyl disulfide to obtain an α-4-methoxybenzylthio compound (this compound, after optional hydrolysis, can be condensed with a compound represented by the general formula (V) or B-(V)), and reacting the α-4-methoxybenzylthio compound with mercuric trifluoroacetate to obtain a corresponding mercapto compound while removing the 4-methoxybenzyl group (see, E. M. Gordon et al., J. Medicinal Chemistry, 31, 2199-2211, 1988).

Next, the biological activities of the present invention are described.

The compounds of the present invention represented by the general formula (I) can activate, for example, macrophages which are immunocompetent cells. To demonstrate this fact, an ability of the compounds (I) to activate macrophages was measured.

The phagocytic ability of mouse peritoneal macrophage was determined by using sheep erythrocytes as a foreign substance.

A female $CDF_1$, mouse, 6 weeks old, was intraperitoneally injected with 1.5 ml of 10% proteose peptone (Difco), and after 3 days, was washed intraperitoneally with Hank's solution (HBSS; Nissui) to recover peritoneal macrophages. The recovered cell suspension was centrifuged at 1000 rpm for 10 minutes, and the precipitated cells were dispersed in RPMI-1640 medium (Nissui) containing 10% fetal bovine serum at a concentration of $4 \times 10^5$ cells/ml. Then, 0.5 ml of the dispersion was added to each well (Ø16 mm) of a 24-well plastic plate, and the plate was incubated in 5% $CO_2$ at 37°C for 3 hours to adhere the macrophages on the plate. Non-adhered cells were washed away with HBSS, and fresh RPMI-1640 containing 10% fetal bovine serum was added to the wells. Next, a peptide to be tested, dissolved in RPMI-1640 containing 10% fetal bovine serum, was added to the wells, and the plate was incubated at 37°C for 15 minutes. Then, $10^6$ sheep red blood cells (SRBC) treated with a anti-sheep red blood cell serum of rabbit were added to each well, and the plate was incubated at 37°C for 45 minutes. Sheep red blood cells which had not been phagocytized by macrophages were hemolyzed with 17 mM Tris-HCl (pH 7.6) containing 0.83% ammonium chloride, and remaining cells were fixed with phosphate-buffered physiological saline (PBS) containing 0.5% glutalaldehyde. The fixed cells were microscopically photographed, and a ratio of macrophages which phagocytized the SRBC was calculated. An ability of a peptide to activate the macrophages for their phagocytic activity was expressed by the percentage, taking an ability wherein no peptide was added as 100%. The results are set forth in Table 1.

Note, tuftsin was used as a reference compound

Table 1

| Compound tested | Concentration (μg/ml) | T/C (%) |
|---|---|---|
| Compound of Example 1 | 0.0015 | 112 |
| | 0.015 | 144 |
| | 0.050 | 171 |
| | 0.150 | 163 |
| Compound of Example 2 | 0.0015 | 126 |
| | 0.015 | 145 |
| | 0.050 | 160 |
| | 0.150 | 166 |
| Reference compound (Tuftsin) | 0.005 | 91 |
| | 0.015 | 137 |
| | 0.050 | 105 |
| | 0.150 | 83 |
| Control | - | 100 |

As shown in Table 1, an effective concentration range of the reference compound, tuftsin, was narrow, and the reference compound showed T/C 137% at the optimum concentration 0.015 μg/ml in the present experiment. On the other hand, a concentration range for activation of macrophages of the compounds of Examples 1 and 2 was very wide; and the compound of Example 1 showed T/C 144% at a concentration of 0.015 μg/ml and T/C 171% at 0.05 μg/ml; and the compound of Example 2 showed T/C 145% at 0.015 μg/ml, and T/C 166% at 0.15 μg/ml.

As seen from the above, the experiment for an ability of the present compound (I) to activate macrophages for their phagocytic action proved that the present compounds exhibit a remarkable macrophage-activating action in a wide concentration range. In addition it was proved that the activation was due to a direct action of the compound on macrophages. This is important when considering the mechanism of action of the present compounds on the immune deficiency syndrome.

The compounds of the present invention represented by the general formula (I) are able to stimulate the blastogenesis of mouse spleen cells by lipopolysaccharide (LPS) or concanavalin A (Con A).

In order to demonstrate this fact the following experiments were carried out.

Assay method: The sample to be tested was dissolved in phosphate-buffered saline (PBS) at a concentration of 10 mg/ml, and then diluted with a cell culture medium (10mM HEPES-RPMI-1640). 50μl each of the diluted sample solution was added to 96-well cell culture plates. Then, 50μl of 10μg/ml Con A solution, or 50μg/ml LPS solution, was added to each wells. Spleen cells were obtained from five weeks old female CDF, mice and dispersed at a concentration of $5x10^6$ cells/ml with 5% FCS -10mM HEPES-RPMI-1640 medium. 100μl of the cell suspension was added to each wells.

The cell culture plates were incubated under 5% $CO_2$ at 37°C for two days. 2μCi of 3H-Thy-midine was added to each wells and further incubated under 5% $CO_2$ at 37°C for one day.

After incubation, cells were collected on a filter paper set on a filter-unit. The radio-isotope counts incorporated into the 5% trichloroacetic acid insoluble fractions were measured by a liquid scintillation counter. Control experiments without a test sample were also performed. Results were expressed in terms of treated/control (T/C)%.

Table 2

| Test sample | Concentration (μg/ml) | T/C (%) LPS | T/C (%) Con A |
|---|---|---|---|
| Example 8 | 2.5 | 94.7 | 95.1 |
| | 25 | 135 | 113 |
| | 250 | 151 | 124 |
| Example 11 | 2.5 | 96.5 | 103 |
| | 25 | 112 | 106 |
| | 250 | 123 | 123 |
| Example 12 | 2.5 | 92.4 | 97.7 |
| | 25 | 104 | 100 |
| | 250 | 120 | 111 |

The reference compound, tuftsin, did not show any stimulation in the above experiments.

In addition, the present compounds (I) have a strong inhibiting activity on leucine aminopeptidase (microsomal) [EC 3.4.11.2]. To prove this, the inhibitory activity of the present compounds to the enzyme was measured as follows.

| | |
|---|---|
| Buffer solution: | 0.1 M Tris-HCl (pH 7.6) |
| Substrate: | 2 mM L-phenylalanyl-β-naphthylamide in 20% DMSO aqueous solution |
| Enzyme: | Leucine aminopeptidase, microsomal [EC 3.4.11.2] (Sigma, from porcine kidney, Lot 57F-8118; when used, the commercial enzyme preparation is dissolved in the buffer solution so that 0.05 ml of the diluted enzyme solution releases about 25 nmoles of β-naphtylamine for 30 minutes) |

| | |
|---|---|
| Garnet reagent: | 1 mg/ml Fast Garnet GBC diazonium salt in 10% Tween 20/1 M acetate buffer (pH 4.2) |
| Method of measurement: | To a test tube (15 x 100 mm), were added 0.25 ml of the substrate solution, 0.5 ml of the buffer solution and 0.2 ml of a sample or water, and after an incubation at 37°C for 3 minutes, 0.05 ml of leucine aminopeptidase [EC 3.4.11.2] solution was added thereto. The reaction was carried out at 37°C for 30 minutes, and after the reaction, 1.0 ml of the Garnet reagent was added to the reaction mixture, the reaction mixture was allowed to stand at a room temperature for 15 minutes, and the absorbance of the mixture was measured at 525 nm. |

According to the above-mentioned procedure, compounds of Examples 1 and 2, as well as reference compounds, bestatin and tuftsin (both from Peptide Institute, INC.) were assayed to determine their enzyme inhibitory activity. The results are set forth in Table 2.

Table 2

| Compound tested | Concentration for 50% inhibition ($IC_{50}$ , μg/ml) |
|---|---|
| Compound of Example 1 | 0.065 |
| Compound of Example 2 | 0.008 |
| Reference compound | |
| Bestatin | 0.3 |
| Tuftsin | 31 |

Note, Bestatin is used as a therapeutic agent for cancer therapy, and is known as an inhibitor of leucine aminopeptidase [EC 3.4.11.1] and aminopeptidase B [EC 3.4.11.6] (Suda et al., Arch. Biochem. Biophys., 177, 196-200, 1976).

As shown in Table 2, the present compounds of the general formula (I) are strong inhibitors of leucine aminopeptidase [EC 3.4.11.2], and therefore, are resistant to the hydrolysis thereof by the enzyme.

The present compounds can be used as pharmaceuticals for the therapy of the immune deficiency syndrome, including cancers, in various administration forms, for example, formulations for external or oral administration, such as tablets, capsules, powders, granules and liquids, and formulations for parenteral administration such as sterilized liquid formulations such as solution and suspensions.

Although the compound as such can be formulated as a solid formulation such as tablets , capsules, granules or powders, it is preferably formulated together with suitable additives. These additives include sugars such as lactose and glucose, starch such as corn starch, wheat starch and rice starch, fatty acids such as stearic acid, inorganic salts such as aluminum magnesium silicate and anhydrous calcium phosphate, synthetic polymers such as polyvinyl-pyrrolidone and polyalkylene glycol, salts of fatty acids such as calcium stearate and magnesium stearate, alcohols such as stearyl alcohol and benzylalcohol, synthetic cellulose derivatives such as methyl cellulose, carboxymethyl cellulose, ethyl cellulose and hydroxypropyl methyl cellulose, and other conventional additives such as water, gelatin, talc, vegetable oil, gum arabic, and the like.

The solid formulations such as tablets, capsules, granules and powders contain the present compound as an active ingredient in an amount of 0.1 to 100%, preferably 5 to 100% by weight relative to the total weight of the formulation.

The liquid formulations contain water, alcohols, or oily liquids such as soybean oil, peanut oil or sesame oil, and may be in the form of a suspension, syrup or injectable liquid.

For parenteral formulations for intramuscular, intravenous or subcutaneous administration, a suitable solvent is used, for example, injectable distilled water, lidocaine hydrochloride aqueous solution (for intramuscular injection), physiological saline, glucose aqueous solution, ethanol, injectable liquid for an intravenous injection (for example, an aqueous solution of citric acid and sodium citrate), or electrolyte solution (for intravenous infusion and injection), or a mixture thereof.

An injectable formulation may be a liquid ready for direct use, or a solid formulation which is an active ingredient as such or a mixture of an active ingredient and additives, which solid formulation is dissolved shortly before use. These invectable liquids generally contain 0.1 to 10%, preferably 1 to 5% by weight of an active ingredient.

The practically preferable dose of the present compound depends on a particular compound used as an active ingredient, the nature of the components used in a composition, the frequency of administration, a particular site to be treated, the host and the tumors. For example, a unit dose for an adult per day is 1 to 500 mg for oral administration, and 1 to 100 mg per day for a parenteral, preferably an intravenous, injection. Note, the pharmaceutical preparation is usually administered 1 to 5 times per day, although this depends on the application routes and the condition of the patient. The pharmaceutical preparation also may be intermittently, administered, for example, once in two days or once in three days.

Examples

The present invention will be further illustrated by but is by no means limited to the following examples.

Example 1 (2S,3R)-AHPA-cyclo(-$N^{\epsilon}$-(S)-lysyl-(S)-prolyl-(S)-arginyl-)

(1) (4R,5S)-3-tert-butoxycarbonyl-2,2-dimethyl-4-phenylmethyl-5-oxazolidine carboxylic acid

(a) 10 g of D-phenylalanine was suspended in 180 ml of dioxane/water (2:1), and 20.2 ml of 3 N sodium hydroxide aqueous solution added thereto under ice cooling. Next, 4.5 ml of a solution of 14.5 g of di-tert-butyl dicarbonate in tetrahydrofuran (THF) was added to the reaction mixture and the mixture stirred overnight. After distilling the solvent from the reaction mixture, 5% potassium bisulfate solution was added to the residue to adjust the pH value to 2 to 3. The reaction mixture was extracted three times with ethyl acetate, the ethyl acetate extracts combined, and the combined extract washed with water and a saturated sodium chloride aqueous solution. The ethyl acetate extract was dried over anhydrous magnesium sulfate, and the solvent distilled off under reduced pressure to obtain 17 g of N-tert-butoxycarbonyl-D-phenylalanine as a colorless oil.

Rf = 0.56 (chloroform/methanol/acetic acid = 10 : 1 : 0.1)

(b) 15 g of the compound obtained in the step (a) was dissolved in 45 ml of DMF, and to the resulting solution were added 14.3 g of sodium bicarbonate and 3.9 ml of methyl iodide while stirring at room temperature, and the stirring was continued overnight at room temperature in the dark. An inorganic substance was filtered off from the reaction mixture, and after an addition of 100 ml of water, the filtrate was extracted with ethyl acetate. The ethyl acetate layer was separated and sequentially washed with a 5% sodium thiosulfate aqueous solution, a 5% sodium bicarbonate aqueous solution and a saturated sodium chloride aqueous solution, and after drying the reaction mixture over anhydrous magnesium sulfate, the solvent was distilled off therefrom to obtain 15.5 g of N-tert-butoxycarbonyl-D-phenylalanine methyl ester as a colorless oil.

Rf = 0.53 (n-hexane/ethyl acetate = 3:1)

(c) 8 g of the compound obtained at step (b) was dissolved in 50 ml of dried toluene, the solution cooled to -78°C under argon gas atmosphere, and 43 ml of 1 M diisobutyl aluminium hydride solution in toluene dropwise added thereto over 15 minutes. The reaction mixture was stirred at the same temperature for 30 minutes, and after dropwise adding 85.8 ml of 1 M vinyl magnesium bromide solution in THF over one hour, warmed to 0°C, and further stirred overnight at the same temperature. Then to the reaction mixture were added 1.6 ml of methanol and 300 ml of 10% citric acid aqueous solution, and the whole was extracted with ethyl acetate. The ethyl acetate layer was separated, washed with a saturated sodium chloride aqueous solution, dried over anhydrous magnesium sulfate, the solvent distilled off under reduced pressure, and the residue was subjected to silica gel column chromatography using 180 g of Kiesel Gel 60 and eluted with n-hexane/ethyl acetate (7:2), to obtain 2.7 g of (3R,4R)-4-tert-butoxycarbonylamino-5-phenyl-1-penten-3-ol as a colorless crystal.

Rf = 0.29 (n-hexane/ethyl acetate = 3:1)

(d) 1 g of the compound obtained at step (c) was dissolved in 2.6 ml of 2,2-dimethoxypropane, and after adding 62 mg of anhydrous p-toluenesulfonic acid, the reaction mixture was stirred over night at room temperature. The reaction mixture was neutralized with sodium bicarbonate, and stirred for 20 minutes, and an inorganic substance was filtered off. The filtrate was evaporated under reduced pressure, and the residue was subjected to silica gel column chromatography using 75 g of Kiesel Gel 60 and eluted with n-hexane/ethyl acetate (15:1), to obtain 847 mg of (4R,5R)-3-tert-butoxycarbonyl-2,2-dimethyl-5-ethenyl-4-phenylmethyloxyazolidine as a colorless oil.

Rf = 0.56 (n-hexane/ethyl acetate = 6:1)

(e) 200 mg of the compound obtained at step (d) was dissolved in 2 ml of dioxane, and after adding 2 ml of a solution of 8 mg of osmium tetraoxide in dioxane at room temperature, the reaction mixture was stirred for 15 minutes in the dark. Then to the reaction mixture was added 0.65 ml of water, and 3.7 ml of an aqueous solution of 269 mg of sodium periodite was added dropwise thereto over 35 minutes. The reaction mixture was stirred for one hour at a room temperature in the dark, and filtered to eliminate an inorganic substance. The filtrate was diluted with ether and washed with a 5% sodium sulfide aqueous solution, the mixture further washed with a saturated sodium chloride aqueous solution, and dried over anhydrous magnesium sulfate. The solvent was evaporated off, and the residue was subjected to silica gel chromatography using 7 g of Kiesel Gel 60 and eluted with n-hexane/ethyl acetate (6:1), to obtain 166.5 mg of (4R,5S)-3-tert-butoxycarbonyl-2,2-dimethyl-5-formyl-4-phenylmethyloxazolidine as a colorless solid.

Rf = 0.38 (n-hexane/ethyl acetate = 3:1)

(f) 135.7 mg of the formyl compound obtained in the step (e) was dissolved in 6.8 ml of acetone, and after adding 300 µl of 2.6 M Jones reagent at -20°C with stirring, the mixture was stirred for one hour. Then, to the reaction mixture was added isopropanol, to consume excess amount of the reagent, and chromium salt was filtered off. The filtrate was diluted with ethyl acetate, washed with water and a saturated sodium chloride aqueous solution, the mixture was dried over anhydrous magnesium sulfate, and the solvent evaporated off, to obtain 131 mg of (4R,5S)-3-tert-butoxycarbonyl-2,2-dimethyl-4-phenylmethyl-5-oxazolidine carboxylic acid as a white solid.

Rf = 0.15 (n-hexane/ethyl acetate = 3:1)

(2) Cyclo(-$N^{\varepsilon}$-(S)-lysyl-(S)-prolyl-$N^{\omega}$-mesitylsulfonyl-(S)-arginyl-)·trifluoroacetate

(a) 198 mg of N-tert-butoxycarbonyl-$N^{\omega}$-mesitylsulfonyl-L-arginine·ethyl acetate·monohydrate was dissolved in 0.5 ml of DMF, and after adding 35 mg of sodium bicarbonate and 46 µl of benzylbromide, the reaction mixture was stirred overnight. The reaction mixture was diluted with ethyl acetate, and washed with water and a saturated sodium chloride aqueous solution, and after drying over anhydrous sodium sulfate, the solvent was evaporated off and the residue was subjected to silica gel column chromatography using 5 g of Kiesel Gel 60 and eluted with chroloform/methanol = 20:1, to obtain 160 mg of N-tert-butoxycarbonyl-$N^{\omega}$-mesitylsulfonyl-L-arginine benzyl ester as a colorless oil.

Rf = 0.34 (chloroform/methanol = 20:1)

(b) 160 mg of the compound obtained at step (a) was dissolved in 0.5 ml of dichloromethane, and after adding 0.18 g of p-toluenesulfonic acid·mono-hydrate under ice cooling, the reaction mixture was warmed to room temperature and stirred for 3 hours. A sodium bicarbonate aqueous solution was added to the reaction mixture and extracted with ethyl acetate, and the ethyl acetate layer separated and washed with water and saturated sodium chloride aqueous solution. The reaction mixture was then dried over anhydrous sodium sulfate and the solvent evaporated off, to obtain 130 mg of $N^{\omega}$-mesitylsulfonyl-L-arginine benzyl ester as a pale yellow oil.

Rf = 0.37 (chroloform/methanol = 10:1)

(c) 68.7 mg of N-tert-butoxycarbonyl-L-proline was dissolved in 0.3 ml of DMF, and after adding 54 mg of HOBt·$H_2O$, 78 mg of DCC and 130 mg of the compound obtained in the step (b), the reaction mixture was warmed to room temperature and stirred overnight. The reaction mixture was filtered to eliminate insoluble material, diluted with ethyl acetate, and washed with 1 N hydrochloric acid, water, saturated sodium bicarbonate aqueous solution, water and saturated sodium chloride aqueous solution. The mixture was then dried over anhydrous magnesium sulfate, the solvent evaporated off, and the residue was purified by silica gel chromatography (Kiesel Gel 60, 5 g; elution with chloroform/methanol = 50:1), to obtain 159.4 mg of N-tert-butoxycarbonyl-L-prolyl-$N^{\omega}$-mesitylsulfonyl-L-arginine benzyl ester as a colorless amorphous substance.

Rf = 0.40 (chloroform/methanol = 20:1)

(d) 158 mg of the compound obtained in the step (c) was treated by the same procedure as described in step (b), to obtain 90 mg of L-prolyl-$N^{\omega}$-mesityl-sulfonyl-L-arginine benzyl ester as a colorless amorphous.

Rf = 0.07 (chloroform/methanol = 10:1)

(e) 90 mg of the compound obtained in the step (d) and 56.4 mg of $N^{\varepsilon}$-benzyloxycarbonyl-N-tert-butoxycarbonyl-L-lysine were coupled by the DCC-HOBt method according to the same procedure as described in step (c), and the product purified by silica gel column chromatography (Kiesel Gel 60, 4.5 g; elution with chloroform/methanol = 20:1), to obtain 134 mg of N-tert-butoxycarbonyl-$N^{\varepsilon}$-benzyloxycarbonyl-L-lysyl-L-prolyl-$N^{\omega}$-mesitylsulfonyl-L-arginine benzyl ester as a colorless amorphous substance.

Rf = 0.48 (chloroform/methanol = 10:1)

(f) 110 mg of the compound obtained at step (e) was dissolved in 1 ml of ethanol, and a hydrogenation was carried out using a catalyst, palladium black, under an atmospheric pressure at room temperature. The catalyst was filtered off and the filtrate concentrated under reduced pressure, to obtain 77.0 mg of N-tert-butoxycarbonyl-L-lysyl-L-prolyl-$N^{\omega}$-mesitylsulfonyl-L-arginine as a white solid.

Rf = 0.45 (n-butanol/acetic acid/water = 4:1:1)

(g) 21.3 mg of $HOBt{\cdot}H_2O$ and 25.9 mg of EDC·HCl were dissolved in 5 ml of DMF, and to the solution was added dropwise, with ice cooling, 15 ml of a DMF solution of 77 mg of the compound obtained at step (f), for 1.5 hours. The reaction mixture was warmed to room temperature, stirred overnight, and concentrated under reduced pressure, and after adding water, the residue was extracted with ethyl acetate. The ethyl acetate layer was separated and sequentially washed with 1 N hydrochloric acid, water, saturated sodium bicarbonate aqueous solution, water, and saturated sodium chloride aqueous solution, and after drying over anhydrous sodium sulfate, the solvent was evaporated off, and the residue was purified by silica gel chromatography (Kiesel Gel 60, 8 g; elution with chloroform/methanol = 20:1), to obtain 36.8 mg of N-tert-butoxycarbonyl-cyclo(-$N^{\varepsilon}$-L-lysyl-L-prolyl-$N^{\omega}$-mesitylsulfonyl-L-arginine-) as a white solid.

Rf = 0.22 (chloroform/methanol = 10:1)

(h) 74 mg of the compound obtained at step (g) was dissolved in 0.5 ml of dichloromethane, and after adding 0.3 ml of trifluoroacetic acid, the reaction mixture was stirred at a room temperature for 3 hours. The solvent and the reagent were evaporated off from the reaction mixture, to obtain 75.2 mg of cyclo(-$N^{\varepsilon}$-(S)-lysyl-(S)-prolyl-$N^{\omega}$-mesitylsulfonyl-(S)-arginyl-)·tryfluoroacetate as a white solid.

(3) <u>(2S,3R)-AHPA-cyclo(-$N^{\varepsilon}$-(S)-lysyl-(S)-prolyl-(S)-arginyl-)·2 acetate</u>

(a) 36.9 mg of the compound obtained in (1) and 75.2 mg of the compound obtained in (2), which was neutralized with 16 µl of triethylamine, were coupled by DCC-HOBt method, and the product was purified by silica gel chromatography (Kiesel Gel 60, 10 g; elution with chloroform/methanol = 10:1), to obtain 61.4 mg of a coupling product as a white solid.

Rf = 0.44 (chloroform/methanol = 10:1)

(b) To 61.4 mg of the compound obtained at step (a) were added 0.4 ml of methanesulfonic acid and 0.04 ml of anisole, and the mixture was stirred for 2 hours at room temperature. Ethyl ether was added to the reaction mixture, which was then decanted to eliminate the added ethyl ether. After adding water to the residue, the solution was passed through 50 ml of Dowex 1-X2 (acetate form), and water passed through the resin. The passed solution was collected, water was evaporated off, and the residue dissolved in a small amount of methanol. The solution was applied to a Sephadex LH-20 column (Ø1.2 cm x 110 cm), and elution was carried out using methanol, to obtain 10.3 mg of the title compound.

FAB-MS: 559 $[M + H]^+$;

$^1$H-NMR (DMSO-$d_6$, δ ppm): 8.01 (1H, brs), 7.82 (1H, d, J = 9.2 Hz), 7.76 (1H, d, J = 7.6 Hz), 7.42 (4H, brs), 7.10 - 7.30 (7H, m), 4.58 (1H, m), 4.12 (2H, m), 3.77 (2H, m), 3.68 (1H, d, J = 2.5 Hz), 2.85 - 3.20 (5H, m), 2.71 (1H, dd, J = 6.7, 13.2 Hz), 2.48 (1H, m), 2.12 (1H, m), 2.02 (1H, m), 1.78 (6H, s), 1.10 - 1.90 (12H, m);

IR (KBr, $cm^{-1}$): 3382, 1659, 1536, 1452.

<u>Example 2 (2S,3R)-AHCA-cyclo(-$N^{\varepsilon}$-(S)-lysyl-(S)-prolyl-(S)-arginyl-)</u>

(1) <u>(4R,5S)-3-tert-butoxycarbonyl-4-cyclohexylmethyl-2,2-dimethyl-5-oxazolidine carboxylic acid</u>

(a) 14.8 g of D-phenylalanine was dissolved in 300 ml of acetic acid/water (1:1), and after adding 1.5 g of platinum oxide, the reaction mixture was subjected to hydrogenation in an autoclave at room temperature under 70 kg/$cm^2$ hydrogen pressure. After 7 hours, the reaction was terminated, the catalyst was filtered off, and the filtrate was concentrated. The residue was crystallized from ethanol/water and the crystal recovered by filtration and dried, to obtain 14.9 g of D-cyclohexylalanine as a white solid.

Rf = 0.44 (chloroform/methanol/acetic acid = 6:3:1)

(b) The compound obtained at step (a) was treated according to the same procedure as described in Example 1, (a) to (f), to obtain (4R,5S)-3-tert-butoxycarbonyl-4-cyclohexylmethyl-2,2-dimethyl-5-oxazolidine carboxylic acid as a white solid.

Rf = 0.07 (n-hexane/ethyl acetate = 4:1)

(2) (2S,3R)-AHCA-cyclo(-$N^{\varepsilon}$-(S)-lysyl-(S)-prolyl-(S)-arginyl-)·2 acetate

(a) 67.5 mg of the compound obtained at step (1) and 130 mg of cyclo(-$N^{\varepsilon}$-(S)-lysyl-(S)-prolyl-$N^{\omega}$-mesitylsulfonyl-(S)-arginyl-)·trifluoroacetate obtained in Example 1 (2) were coupled using the DCC-HOBt method according to the same procedure as described in Example 1 (3), and the product purified by silica gel column chromatography (Kiesel Gel 60, 13 g; elution chloroform/methanol = 10:1) to obtain 60.7 mg of a coupling product as a white solid.

Rf = 0.42 (chloroform/methanol = 10:1)

(b) to 55.7 mg of the compound obtained at step (a) were added 0.4 ml of methanesulfonic acid and 0.02 ml of anisole, and the mixture was stirred at room temperature for 3 hours. The reaction product was purified according to the same procedure as described in Example 1 (3), to obtain 35.0 mg of the title compound.

FAB-MS: 565 $[M + H]^+$;

$^1$H-NMR (DMSO-$d_6$, δ ppm): 8.05 (1H, brs), 7.83 (1H, d, J = 9.4 Hz), 7.77 (1H, brd, J = 7.2 Hz), 7.49 (4H, brs), 7.15 (1H, brt, J = 5.2 Hz), 4.58 (1H, m), 4.13 (2H, m), 3.79 (2H, m), 3.66 (1H, brs), 2.85 - 3.25 (5H, m), 2.15 (1H, m), 2.04 (1H, m), 1.79 (6H, s), 0.70 - 1.90 (25H, m);

IR (KBr, $cm^{-1}$): 3364, 2926, 2854, 1671, 1641, 1563, 1455, 1410.

Example 3
(2S,3R)-3-amino-2-hydroxy-4-(4-hydroxy-phenyl)butanoyl-cyclo(-$N^{\varepsilon}$-(S)-lysyl-(S)-prolyl-(S)-arginyl-)

(1)
(4R,5S)-3-tert-butoxycarbonyl-4-{4-(2,6-dichlorophenylmethyloxy)phenylmethyl}-2,2-dimethyl-5-oxazolidine carboxylic acid

$N^{\alpha}$-tert-butoxycarbonyl-O-(2,6-dichlorobenzyl)-D-tyrosine was treated according to the same procedure as described in Example 1, (b) to (f), to obtain (4R,5S)-3-tert-butoxycarbonyl-4-{4-(2,6-dichlorophenylmethyloxy}phenylmethyl}-2,2-dimethyl-5-oxazolidine carboxylic acid as a white solid.

Rf = 0.66 (ethyl acetate/methanol/acetic acid = 10:0.5:1)

(2) (2S,3R)-3-amino-2-hydroxy-4-(4-hydroxy)phenylbutanoyl-cyclo(-$N^{\varepsilon}$-(S)-lysyl-(S)-prolyl-(S)-arginyl-)·2 acetate

(a) 45.9 mg of the compound obtained in the step (1) and 61.3 mg of cyclo(-$N^{\varepsilon}$-(S)-lysyl-(S)-prolyl-$N^{\omega}$-mesitylsulfonyl-(S)-arginyl-)·trifluoroacetate obtained in Example 1 (2) were coupled using the DCC-HOBt method according to the same procedure as described in Example 1 (3) (a), and the product was purified by silica gel chromatography (Kiesel Gel 60, 3 g; elution with ethyl acetate/methanol = 20:1) to obtain 73 mg of a coupling product as a white solid.

Rf = 0.42 (ethyl acetate/methanol = 10:1)

(b) To 69.8 mg the compound obtained in the step (a) were added 0.5 ml of methanesulfonic acid and 0.03 ml of anisole, and the mixture was stirred at room temperature for 2 hours. The product was purified according to the same procedure as described in Example 1 (3) to obtain 30.5 mg of the title compound.

FAB-MS: 575 $[M + H]^+$;

$^1$H-NMR (DMSO-$d_6$, δ ppm): 9.15 (1H, brs), 7.81 (1H, d, J = 8.7 Hz), 7.77 (1H, d, J = 9.3 Hz), 7.56 (1H, brs), 6.98 (2H, d, J = 8.0 Hz), 6.66 (2H, d, J = 8.0 Hz), 4.57 (1H, m), 4.12 (2H, m), 3.77 (2H, m), 3.68 (1H, d, J = 3.1 Hz), 2.85 - 3.20 (5H, m), 2.60 (1H, m), 2.38 (1H, m), 2.15 (1H, m), 2.01 (1H, m), 1.10 - 1.95 (15H, m);

IR (KBr, $cm^{-1}$): 3382, 1665, 1524, 1455.

Example 4 (2S,3R)-3-amino-2-hydroxybutanoyl-cyclo(-$N^{\varepsilon}$-(S)-lysyl-(S)-prolyl-(S)-arginyl-)

(1) (4R,5S)-3-tert-butoxycarbonyl-2,2,4-trimethyl-5-oxazolidine carboxylic acid

3.07 g of D-alanine was treated according to the same procedure as described in Example 1, (a) to (f), to obtain 1.72 g of (4R,5S)-3-tert-butoxy-carbonyl-2,2,4-trimethyl-5-oxazolidine carboxylic acid as a pale yellow oil.

Rf = 0.18 (chloroform/methanol = 10:1)

(2) (2S,3R)-3-amino-2-hydroxybutanoyl-cyclo(-$N^{\varepsilon}$-(S)-lysyl-(S)-prolyl-(S)-arginyl-)·2 acetate

(a) 62.4 mg of the compound obtained at step (1) and 173.1 mg of cyclo(-$N^{\varepsilon}$-(S)-lysyl-(S)-prolyl-$N^{\omega}$-mesitylsulfonyl-(S)-arginyl-)·trifluoroacetate salt, which was neutralized with 36 μl of triethylamine, were coupled by the DCC-HOBt method, and the product purified by silica gel column chromatography (Kiesel Gel 60, 16 g; elution with chloroform/methanol = 20:1) to obtain 51.7 mg of a coupling product as a white solid.

Rf = 0.36 (chloroform/methanol = 10:1)

(b) to 45.5 mg of the compound obtained at step (a) were added 0.5 ml of methanesulfonic acid and 0.02 ml of anisole, and the mixture was stirred at a room temperature for 3 hours. The product was purified according to the same procedure as described in Example 1 (3) (b), and crystallized from ethyl ether/ethyl acetate to obtain 34.0 mg of the title compound.

FAB-MS: 483 $[M + H]^+$

$^1$H-NMR (DMSO-$d_6$, δ ppm): 8.30 (1H, brs), 7.84 (2H, m), 7.42 (4H, brs), 7.12 (1H, brs), 4.58 (1H, m), 4.12 (2H, m), 3.78 (2H, m), 3.65 (1H, brs), 2.88 - 3.25 (5H, m), 2.16 (1H, m), 2.05 (1H, m), 1.79 (6H, s), 1.05 - 1.90 (12H, m), 0.95 (3H, brd, J = 6.0 Hz).

Example 5 (2S,3R)-AHPA-cyclo(-$N^{\delta}$-(S)-ornithyl-(S)-prolyl-(S)-arginyl-)

(1) Cyclo(-$N^{\delta}$-(S)-ornithyl-(S)-prolyl-(S)-arginyl-)·trifluoroacetic acid

(a) 156 mg of $N^{\delta}$-benzyloxycarbonyl-N-tert-butoxycarbonyl-L-ornithine was dissolved in 0.5 ml of dry DMF, and to the solution were added 70.6 mg of HOBt·$H_2O$, 95.1 mg of DCC and 0.5 ml of a DMF solution of 193 mg of L-prolyl-N -mesitylsulfonyl-L-arginine benzyl ester obtained in Example 1 (2)(d), with stirring and under ice cooling. The reaction mixture was warmed to room temperature, stirred overnight, and filtered to eliminate insoluble matter. The filtrate was diluted with ethyl acetate, and sequentially washed with a 5% potassium bisulfate aqueous solution, 4% sodium bicarbonate aqueous solution, and a saturated sodium chloride aqueous solution. The mixture was then dried over anhydrous magnesium sulfate, the solvent was evaporated under reduced pressure, and the residue was purified by silica gel column chromatography (Kiesel Gel 60, 30 g; elution with ethyl acetate/methanol = 20:1), to obtain 195.3 mg of $N^{\delta}$-benzyloxycarbonyl-N-tert-butoxycarbonyl-L-ornithyl-L-prolyl-$N^{\omega}$-mesitylsulfonyl-L-arginine benzyl ester as a white solid.

Rf = 0.10 (n-hexane/ethyl acetate = 1:4)

(b) 190 mg of the compound obtained at step (a) was hydrogenated according to the same procedure as described in Example 1 (2) (f) to obtain 133 mg of N-tert-butoxycarbonyl-L-ornithyl-L-prolyl-$N^{\omega}$-mesitylsulfonyl-L-arginine as a white solid.

Rf = 0.47 (n-butanol/acetic acid/water = 4:1:1)

(c) 129 mg of the compound obtained at step (b) was treated with EDC-HOBt, and the product was purified by silica gel column chromatography (Kiesel Gel 60, 15 g; elution with ethyl acetate/methanol = 7:1) to obtain 58.9 mg of N-tert-butoxycarbonyl-cyclo(-$N^{\delta}$-L-ornithyl-L-prolyl-$N^{\omega}$-mesitylsulfonyl-L-arginine-) as a white solid.

Rf = 0.10 (ethyl acetate/methanol = 5:1)

(d) 54.5 mg of the compound obtained at step (c) was treated with TFA according to the same procedure as described in Example 1 (2) (h), to obtain a cyclo(-$N^{\delta}$-(S)-ornithyl-(S)-prolyl-$N^{\omega}$-mesitylsulfonyl-(S)-arginyl-)·trifluoroacetate.

Rf = 0.40 (ethyl acetate/methanol = 5:1)

(2) (2S,3R)-AHPA-cyclo(-$N^{\delta}$-(S)-ornithyl-(S)-prolyl-(S)-arginyl-)·2 acetate

(a) 55.8 mg of the compound obtained at step (1) and 28.2 mg of the carboxylic acid obtained in Example 1 (1) were neutralized with triethylamine, and were coupled by the DCC-HOBt method according to the same procedure as described in Example (3) (a), and the product was purified by silica gel column chlomatography (Kiesel Gel 60, 3 g; elution with ethyl acetate/methanol = 10:1) to obtain 52.5 mg of a coupling product as a white solid.

Rf = 0.27 (ethyl acetate/methanol = 10:1)

(b) to 50.1 mg of the compound obtained at (a) were added 0.5 ml of methanesulfonic acid and 28 μl of anisole, and the reaction mixture was stirred at room temperature for 2 hours. The product was purified according to the same procedure as described in Example 1 (3) (b), to obtain 12.1 mg of the title compound as a white solid.

FAB-MS: 545 [M + H]$^+$;
$^1$H-NMR (DMSO-$d_6$, δ ppm): 8.70 (1H, brs), 7.69 (1H, d, J = 7.0 Hz), 7.60 (3H, brs), 7.45 (1H, brs), 7.42 (1H, d, J = 10.5 Hz), 7.13 - 7.32 (5H, m), 4.62 (1H, m), 4.10 (2H, m), 3.87 (1H, m), 3.70 (1H, d, J = 2.9 Hz), 3.62 (1H, m), 3.05 (4H, m), 2.74 (2H, m), 1.75 (6H, s), 1.30 - 2.25 (12H, m);
IR (KBr, $cm^{-1}$): 3412, 1665, 1563, 1536, 1446.

Example 6
(2S,3R)-3-amino-2-hydroxy-4-(4-methoxyphenyl)butanoyl-cyclo-($N^ε$-(S)-lysyl-(S)-prolyl-(S)-arginyl-)

(1) (4R,5S)-3-tert-butoxycarbonyl-2,2-dimethyl-4-[(4-methoxyhenyl)methyl]-5-oxazolidine carboxylic acid

$N^α$-tert-butoxycarbonyl-O-methyl-D-tyrosine was treated according to the same procedure as described in Example 1, (b) to (f), to obtain (4R,5S)-3-tert-butoxy-carbonyl-2,2-dimethyl-4-[(4-methoxyphenyl)methyl]-5-oxazolidine carboxylic acid as a white solid.

Rf = 0.09 (n-hexane/ethyl acetate = 1:1)

(2) (2S,3R)-3-amino-2-hydroxy-4-(4-methoxyphenyl)butanoyl-cyclo(-$N^ε$-(S)-lysyl-(S)-prolyl-(S)-arginyl-)·2 acetate

(a) 40 mg of the compound obtained at step (1) and 74 mg of cyclo(-$N^ε$-(S)-lysyl-(S)-prolyl-$N^ω$-mesitylsulfonyl-(S)-arginyl-)·trifluoroacetate obtained in Example 1 (2) were coupled by the DCC-HOBt method acccrding to the same procedure as described in Example 1 (3)(a), and the product was purified by silica gel column chromatography (Kiesel Gel 60, 4 g; elution with ethyl acetate/methanol = 20:1) to obtain 65.8 mg of a coupling product as a white solid.

Rf = 0.36 (ethyl acetate/methanol = 10:1)

(b) To 31.8 mg of the compound obtained in the step (a) were added 0.3 ml of methanesulfonic acid and 0.03 ml of anisole, and the mixture was stirred at room temperature for 1.5 hours. The product was purified according to the same procedure as described in Example (3) (b), to obtain 19.4 mg of the title compound.
FAB-MS: 589 [M + H]$^+$;
$^1$H-NMR (DMSO-$d_6$, δ ppm): 8.78 (1H, brs), 7.82 (1H, d, J = 9.1 Hz), 7.75 (1H, d, J = 7.9 Hz), 7.67 (4H, brs), 7.10 (2H, d, J = 7.9 Hz), 6.83 (2H, d, J = 7.9 Hz), 4.57 (1H, m), 4.12 (2H, m), 3.75 (2H, m), 3.70 (3H, s), 3.67 (1H, m), 2.85 - 3.20 (5H, m), 2.65 (1H, m), 2.42 (1H, m), 2.14 (1H, m), 2.02 (1H, m), 1.78 (6H, s), 1.10 - 1.90 (12H, m);
IR (KBr, $cm^{-1}$): 3376, 1665, 1560, 1518, 1449, 1410.

Example 7 (2S,3R,4S)-3-amino-2,4-dihydroxy-pentanoyl-cyclo(-$N^ε$-(S)-lysyl-(S)-prolyl-(S)-arginyl-)

(1) (4R,5S)-3-tert-butoxycarbonyl-4-{(1S)-benzyloxy)ethyl}-2,2-dimethyl-5-oxazolidine carboxylic acid

$N^α$-tert-butoxycarbonyl-O-benzyl-D-threonine was treated according to the same procedure as described in Example 1, (b) to (f), to obtain (4R,5S)-3-tert-butoxycarbonyl-4-{(1S)-benzyloxy)ethyl)-2,2-dimethyl-5-oxazolidine carboxylic acid as a white solid.

Rf = 0.65 (ethyl acetate/methanol/acetic acid = 10:0.5:1)

(2) (2S,3R,4S)-3-amino-2,4-dihydroxypentanoyl-cyclo(-$N^ε$-(S)-lysyl-(S)-prolyl-(S)-arginyl-)·2 acetate

(a) 38 mg of the compound obtained in the step (1) and 68 mg of cyclo(-$N^ε$-(S)-lysyl-(S)-prolyl-$N^ω$-mesitylsulfonyl-(S)-arginyl-)·trifluoroacetate obtained in Example 1 (2) were coupled by the DCC-HOBt method according to the same procedure as described in Example 1 (3)(a), and the product was purified by silica gel column chromatography (Kiesel Gel 60, 4 g; elution with ethyl acetate/methanol = 20:1) to obtain 50.6 mg of a coupling product as a white solid.

Rf = 0.46 (ethyl acetate/methanol = 10:1)

(b) To 48 mg of the compound obtained at step (a) were added 0.35 ml of methanesulfonic acid and 0.035 ml of anisole, and the reaction mixture was stirred at room temperature for one hour. The product was purified according to the same procedure as described in Example 1 (3)(b) to obtain 33 mg of the title compound.
FAB-MS: 513 [M + H]$^+$;
$^1$H-NMR (DMSO-$d_6$, δ ppm): 8.45 (1H, brs), 7.79 (1H, m), 7.50 (4H, brs), 7.06 (1H, m), 4.58 (1H, brs), 4.12

(3H, m), 3.74 (3H, m), 2.80 - 3.50 (5H, m), 2.16 (1H, m), 2.02 (1H, m), 1.70 (6H, S), 1.10 - 1.90 (12H, m), 1.05 (3H, d, J = 5.9 Hz);

IR (KBr, $cm^{-1}$): 3412, 1665, 1563, 1536, 1446.

Example 8 $N^{\alpha}$-((2S,3R)-AHCA)-(S)-lysyl-(S)-prolyl-(S)-arginine·2 acetate

(a) 132 mg of N-tert-butoxycarbonyl-$N^{\varepsilon}$-benzyloxy-carbonyl-L-lysyl-L-prolyl-$N^{\omega}$-mesitylsulfonyl-L-arginine benzyl ester obtained in Example 1 (2) (e) was dissolved in 0.6 ml of dichloromethane, 0.4 ml of trifluoroacetic acid was added thereto, and the solution was stirred at room temperature for one hour. The solvent was evaporated off from the reaction mixture under reduced pressure, to obtain 134 mg of a $N^{\varepsilon}$-benzyloxycarbonyl-L-lysyl-L-prolyl-$N^{\omega}$-mesitylsulfonyl-L-arginine benzyl ester·trifluoroacetate salt.

(b) 54.8 mg of (4R,5S)-3-tert-butoxycarbonyl-4-cyclohexylmethyl-2,2-dimethyl-5-oxazolidine carboxylic acid obtained in Example 2 (1)(b) and 134 mg of the compound obtained in step (a) were coupled by the DCC-HOBt method according to the same procedure as described in Example 1, (3) (a), and the product was purified by silica gel column chromatography (Kiesel Gel 60, 6 g; n-hexane/ethyl acetate = 3:10) to obtain 142 mg of a coupling compound as a white solid.

Rf = 0.21 (n-hexane/ethyl acetate = 3:10)

(c) To 82 mg of the compound obtained at step (b) were added 0.6 mg of methanesulfonic acid and 34 μl of anisole, and the reaction mixture was stirred at room temperature for 3.5 hours. The product was purified according to the same procedure as described in Example 1, (3) (b), to obtain 50.4 mg of the title compound as a white solid.

FAB-MS: 583 $[M + H]^+$

Rf = 0.13 (iso-propanol/water/aqueous ammonia = 7:2:1)

Example 9 $N^{\alpha}$-((2S,3R)-3-amino-2-hydroxy-4-(4-methoxyphenyl)butanoyl)-(S)-lysyl-(S)-prolyl-(S)-arginine·2 acetate

(a) 53 mg of (4R,5S)-3-tert-butoxycarbonyl-2,2-dimethyl-4-[(4-methoxyphenyl)methyl]-5-oxazolidine carboxylic acid obtained in Example 6, (1), and 122 mg of $N^{\varepsilon}$-benzyloxycarbonyl-L-lysyl-L-prolyl-$N^{\omega}$-mesitylsulfonyl-L-arginine benzyl ester·trifluoroacetate salt obtained in Example 8 (a) were coupled by the DCC-HOBt method according to the same procedure as described in Example 1 (3) (a), and the product was purified by silica gel column chromatography to obtain 116 mg of a coupling compound as a white solid.

Rf = 0.49 (ethyl acetate)

(b) To 50 mg of the compound obtained at step (a) were added 0.4 ml of methanesulfonic acid and 40 μl of anisol, and the reaction mixture was stirred at room temperature for two hours. The product was purified according to the same procedure as described in Example 1, (3) (b), to obtain 31.3 mg of the title compound as a white solid.

FAB-MS: 607 $[M + H]^+$;

Rf = 0.63 (iso-propanol/water/concentrated aqueous ammonia = 7:3:2)

Example 10 $N^{\alpha}$-((2S,3R)-3-amino-2-hydroxybutanoyl)-(S)-lysyl-(S)-prolyl-(S)-arginine.2 acetate

(a) 60 mg of ((4R,5S)-3-tert-butoxycarbonyl-2,2,4-trimethyl-5-oxazolidine carboxylic acid obtained in Example 4 (1) and 211 mg of $N^{\varepsilon}$-benzyloxycarbonyl-L-lysyl-L-prolyl-$N^{\omega}$-mesitylsulfonyl-L-arginine benzyl ester·trifluoroacetate obtained in Example 8 (a) were coupled by the DCC-HOBt method according to the same procedure as described in Example 1 (3) (a), and the product was purified by silica gel column chromatography (Kiesel Gel 60; elution with chloroform/methanol = 100:1) to obtain 122 mg of a coupling compound as a white solid.

Rf = 0.61 (chloroform/methanol = 10:1)

(b) 121 mg of the compound obtained in the step (a) was dissolved in 1 ml of ethanol, and after adding palladium black thereto, hydrogenation was carried out at ambient temperature and under atmospheric pressure. The catalyst was filtered off, and the filtrate concentrated under reduced pressure. To the resulting solid were added 0.6 ml of methanesulfonic acid and 30 μl of anisole, and the mixture was stirred at room temperature for 2.5 hours. The product was purified according to the same procedure as described in Example 1 (3) (b), to obtain 54 mg of the title compound as a white solid.

FAB-MS: 501 $[M + H]^+$;

Rf = 0.04 (iso-propanol/water/concentrated aqueous ammonia = 7:2:1)

Example 11 $N^{\alpha}$-((2S,3R)-AHCA)-(S)-lencyl-(S)-prolyl- (S)-arginine·acetate

(1) N-[(4R,5S)-3-tert-butoxycarbonyl-4-cyclohexyl-methyl-2,2-dimethyl-5-oxazolidylcarbonyl]-L-leucine

(a) 150mg of (4R,5S)-3-tert-butoxycarbonyl-4-cyclohexylmethyl-2,2-dimethyl-5-oxazolidine carboxylic acid obtained in Example 2, (1)(b), and 173 mg of L-leucine benzylester p-toluenesulfonate were coupled by the DCC-$HOB_t$ method according to the same procedure as described in Example 1, (3)(a), and the product was purified by silica gel column chromatography (Kiesel gel 60, 25g, n-hexane/ethyl acetate = 8:1) to obtain 220mg of a coupling product N-[(4R,5S)-3-tert-butoxycarbonyl-4-cyclohexylmethyl-2,2- dimethyl-5-oxazolidyl-carbonyl]-L-leucine benzyl ester, as a white solid.

Rf = 0.45 (n-hexane/ethyl acetate = 5:1)

(b) 105mg of the compound obtained in step(a) was dissolved in 1.5ml of ethanol and was hydrogenated in the presence of 10% Pd-c under atomospheric pressue at room temperature. After the reaction, the catalyst was removed by filtration and the filtrate was distilled under reduced pressure to obtain 85mg of N-[(4R,5S)-3-tert-butoxycarbonyl-4-cyclohexylmethyl-2,2-dimethyl-5-oxazolidyl-carbonyl]-L-leucine as a white solid.

Rf = 0.32 (chloroform/ethyl acetate/acetic acid = 10:1:0.1)

(2) L-Prolyl-$N^{\omega}$-mesitylsufonyl-L-arginine benzylester·trifluoroacetate

To 1ml of methylene chleride solution of 303mg of the compound obtained in Example 1, (2)(b), was added 1ml of trifluoro-acetic acid and the solution was stirred for one hour at room temperature. The solution was evaporated under reduced pressure to obtain 309mg of L-prolyl-$N^{\omega}$-mesitylsufonyl-L-arginine benzylester· trifluoroacetate as a white solid.

Rf = 0.17 (chloroform/methanol/concentrated ammonia water = 10:1:0.1)

(3) $N^{\alpha}$((2S,3R)-AHCA)-(S)-lencyl-(S)-prolyl-(S)-arginine·acetate

(a) 50.6mg of the compound obtained in step(1) and 79.6mg of the compound obtained in step(2) were , neutralized with 16.9µl of triethylamine and were coupled by the DCC-HOBt method. The coupled product was purified by silica gel column chromatography (Kiesel gel 60, chloroform/methanol = 50:1) to obtain 69.1mg of the purified compound as a white solid.

FAB-MS: 980 $[M+H]^+$

RF = 0.44 (Chloroform/methanol = 20:1)

(b) To 50.2mg of the compound obtained in step(a) was added 0.1ml of methanesulfonic acid and 60µl of anisole and the solution was stirred for two hours at room temperature. 12.0mg of the title compound was obtained by the same procedure described in Example 1, (3)(b), as a white solid.

FAB-MS: 568 $[M+H]^+$

RF = 0.5 (iso-propanol/water/concentrated ammonia water = 7:2:1)

$^1$H-NMR ($CD_3OD$,δppm): 4.56 (1H, m), 4.43 (1H, m), 4.12 - 4.28 (2H, m), 3.44 - 3.60 (3H, m), 3.20 (2H, m), 1.97 (3H, S), 0.9 - 2.4 (30H)

IR (KBr,$cm^{-1}$): 3388, 2926, 1641, 1455, 1407

Example 12 $N^{\alpha}$-(2S,3R)-AHCA-(S)-β-cyclohexyl-alanyl-(S)-prolyl-(S)-arginine·acetate

(a) 100mg of (4R,5S)-3-tert-butoxycarbonyl-4-cyclohexylmethyl-2,2-dimethyl-5-oxazolidine carboxilic acid obtained in Example 2, (1)(b), and 148mg of L-β-cyclohexylalanine benzylester·p-toluenesulfonate were coupled by the same procedure described in Example 1, (3)(a), and the coupled product was purified by silica gel column chromatography (Kiesel gel 60, 20g, n-hexane/ethyl acetate = 8:1) to obtain 116mg of N-[(4R,5S)-3-tert-butoxycarbonyl-4-cyclohexylmethyl-2,2-dimethyl-5-oxazolidylcarbonyl]-L-β-cyclohexyl -alanine benzylester as a white solid.

Rf = 0.49 (n-hexane/ethyl acetate = 5:1)

(b) 115mg of the compound obtained in step(a) was dissolved in 1.5ml of ethanol and was hydrogenated in the presence of 10% Pd-C under atomospheric pressure at room temperature. After the reaction, the catalyst was removed by filtration and the filtrate was evaporated under reduced pressure to obtain 97mg of N-[(4R,5S)-3-tert-butoxycarbonyl-4-cyclohexylmethyl-2.2-dimethyl-5-oxazolidylcarbonyl]-L-β-cyclohexylalanine as a white solid.

Rf = 0.22 (chloroform/ethyl acetate/acetic acid = 10:1:0.1)

(c) 50mg of the compound obtained in step(b) and 73.1mg of the compound obtained in Example 11, (2) were neutralized with 15.5μl of triethylamino and were coupled by the DCC-HOBt method and the coupled product was purified by silica gel column chromatography (Kiesel gel 60, chloroform/methanol = 50:1) to obtain 54.9mg of the purified compound as a white solid.

Rf = 0.37 (Chloroform/methanol = 20:1)

(d) To 53mg of the compound obtained in step(c) was added 0.4ml of methanesulfonic acid and 60μl of anisole and the solution was stirred for two hours at room temperature. The reaction product was purified by the same procedure described in Example 1, (3)(b), to obtain 20.2mg of the title compound as a white solid.

FAB-MS: 608$[M+H]^+$

Rf = 0.63 (iso-propanol/water/concentrated ammonia water = 7:2:1)

$^1$H-NMR ($CD_3OD$,δppm): 4.57 (1H, m), 4.42 (1H, m), 4.12 - 4.28 (2H, m), 3.43 - 3.88 (3H, m), 3.43 - 3.88 (3H, m), 3.20 (2H, m), 1.96 (3H, S), 0.9 - 2.4 (34H)

IR (KBr,$cm^{-1}$): 3400, 2926, 2854, 1647, 1452, 1407

The present compounds activate immunocompetent cells, such as macrophages or polymorphonuclear leukocyte, provide a host-mediated inhibition of the growth of tumors, provide protective effects for infectious diseases such as viral, bacterial and fungal diseases, and exhibit therapeutic effects on autoimmune diseases such as lupus erythematosus, rheumatoid and the like, and therefore, are useful for the production of pharmaceutical preparations for the treatment of these diseases.

## Claims

1. A peptide derivative represented by the formula (I):

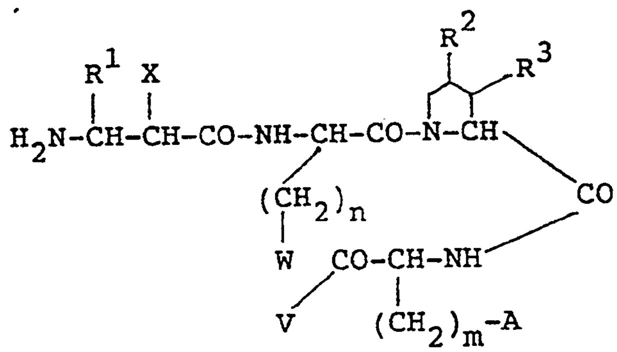

(I)

wherein V represents a hydroxyl group; W represents a hydrogen atom, lower alkyl, cycloalkyl having three to seven carbon atoms, phenyl, amino, mono- or di-lower alkylamino, amidino, or guanidino group; or W and V together form a group of the formula:

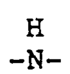 , 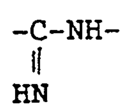 , or 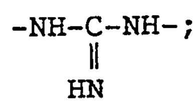 ;

X represents a hydrogen atom, or hydroxy or mercapto group;

$R^1$ represents a hydrogen atom, or a phenyl, p-hydroxyphenyl or lower alkyl group, which lower alkyl group is optionally substituted with one to three of the same or different substituents selected from the group consisting of amino, hydroxy, lower alkoxy, mercapto, lower alkylthio, lower alkylfulfinyl, lower alkylsulfonyl, carboxyl, aryl, heteroaryl, cycloalkyl having three to seven carbon atoms and of bicycloalkyl having eight to ten carbon atoms, wherein the aryl, heteroaryl, cycloalkyl or bicycloalkyl group is optionally substituted with hydroxy, lower alkyloxy, lower alkyl, nitro, amino, mono- or di-loweralkylamino, mercapto, lower alkylthio, lower alkylfulfinyl or with lower alkylsulfonyl;

$R^2$ and $R^3$ are the same and represent a hydrogen atom or a lower alkyl group; or one of $R^2$ and $R^3$ represents a hydrogen atom, and the other represents a hydroxy, lower alkoxy or lower alkyl group; or both $R^2$ and $R^3$ together form a single bond;

A represents an amino, mono- or di- lower alkylamino, amidino, or guanidino group; and
m and n independently represent an integer of 0 to 4;
and pharmaceutically acceptable salts thereof

2. A peptide derivative represented by the formula (II):

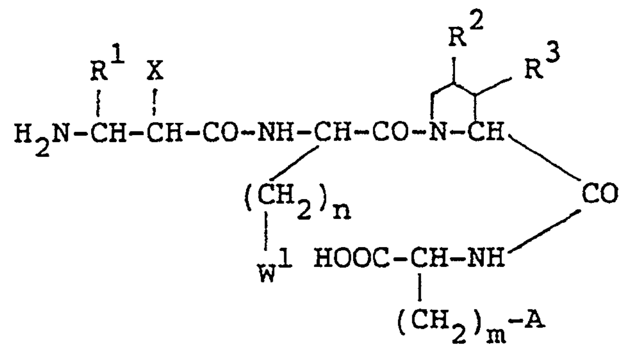

(II)

wherein X represents a hydrogen atom, or hydroxy or mercapto group;
$W^1$ represents a hydrogen atom, lower alkyl, cycloalkyl having three to seven carbon atoms, phenyl, amino, mono- or di-lower alkylamino, amidino, or guanidino group;
$R^1$ represents a hydrogen atom, or a phenyl, p-hydroxyphenyl or lower alkyl group, which lower alkyl group is optionally substituted with one to three of the same or different substituents selected from the group consisting of amino, hydroxy, lower alkoxy, mercapto, lower alkylthio, lower alkylfulfinyl, lower alkylsulfonyl, carboxyl, aryl, heteroaryl, cycloalkyl having three to seven carbon atoms and of bicycloalkyl having eight to ten carbon atoms, wherein the aryl, heteroaryl, cycloalkyl or bicycloalkyl group is optionally substituted with hydroxy, lower alkyloxy, lower alkyl, nitro, amino, mono- or di-loweralkylamino, mercapto, lower alkylthio, lower alkylfulfinyl or with lower alkylsulfonyl;
$R^2$ and $R^3$ are the same and represent a hydrogen atom or a lower alkyl group; or one of $R^2$ and $R^3$ represents a hydrogen atom, and the other represents a hydroxy, lower alkoxy or lower alkyl group; or both $R^2$ and $R^3$ together form a single bond;
A represents an amino, mono- or di- lower alkylamino, amidino, or guanidino group; and
m and n independently represent an integer of 0 to 4;
and pharmaceutically acceptable salts thereof.

3. A peptide derivative represented by the formula (III):

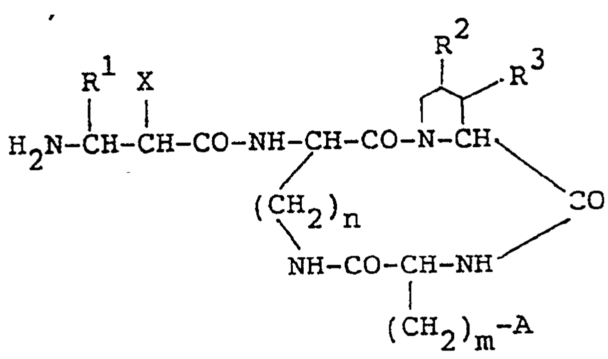

(III)

wherein X represents a hydrogen atom, or hydroxy or mercapto group;
$R^1$ represents a hydrogen atom, or a phenyl, p-hydroxyphenyl or lower alkyl group, which lower alkyl group is optionally substituted with one to three of the same or different substituents selected from the group consisting of amino, hydroxy, lower alkoxy, mercapto, lower alkylthio, lower alkylfulfinyl, lower alkylsulfonyl, carboxyl, aryl, heteroaryl, cycloalkyl having three to seven carbon atoms and of bicycloalkyl having eight to ten carbon atoms, wherein the aryl, heteroaryl, cycloalkyl or bicycloalkyl group is optionally substituted with hydroxy, lower alkyloxy, lower alkyl, nitro, amino, mono- or di-loweralkylamino, mercapto, lower alkylthio, lower alkylfulfinyl or with lower alkylsulfonyl;
$R^2$ and $R^3$ are the same and represent a hydrogen atom or a lower alkyl group; or one of $R^2$ and $R^3$ repre-

sents a hydrogen atom, and the other represents a hydroxy, lower alkoxy or lower alkyl group; or both $R^2$ and $R^3$ together form a single bond;
A represents an amino, mono- or di-lower alkylamino, amidino, or guanidino group; and
m and n independently represent an integer of 0 to 4;
and pharmaceutically acceptable salts thereof.

**4.** A peptide derivative selected from the group consisting of:
AHPA-cyclo(-$N^{\varepsilon}$-(S)-lysyl-(S)-prolyl-(S)-arginyl-);
AHCA-cyclo(-$N^{\varepsilon}$-(S)-lysyl-(S)-prolyl-(S)-arginyl-);
3-amino-2-hydroxy-4-(4-hydroxy-phenyl)butanoyl-cyclo(-$N^{\varepsilon}$-(S)-lysyl-(S)-prolyl-(S)-arginyl-);
3-amino-2-hydroxybutanoyl-cyclo(-$N^{\varepsilon}$-(S)-lysyl-(S)-prolyl-(S)-arginyl-);
AHPA-cyclo(-$N^{\delta}$-(S)-ornithyl-(S)-prolyl(S)-arginyl-);
3-amino-2-hydroxy-4-(4-methoxyphenyl)butanoyl-cyclo(-$N^{\varepsilon}$-(S)-lysyl-(S)-prolyl-(S)-arginyl-);
3-amino-2,4-dihydroxypentanoyl-cyclo(-$N^{\varepsilon}$-(S)-lysyl-(S)-prolyl-(S)-arginyl-);
$N^{\alpha}$-AHCA-(S)-lysyl-(S)-proyl-(S)-arginine;
$N^{\alpha}$-(3-amino-2-hydroxy-4-(4-methoxyphenyl)butanoyl)-(S)-lysyl-(S)-prolyl-(S)-arginine;
$N^{\alpha}$-(3-amino-2-hydroxybutanoyl)-(S)-lysyl-(S)-prolyl-(S)-arginine;
$N^{\alpha}$-AHCA-(S)-leucyl-(S)-prolyl-(S)-arginine; and
$N^{\alpha}$-AHCA-(S)-β-cyclohexylalanyl-(S)-prolyl-(S)-arginine; and
a pharmaceutically acceptable salt thereof.

**5.** A peptide derivative selected from the group consisting of:
(2S,3R)-AHPA-cyclo(-$N^{\varepsilon}$-(S)-lysyl-(S)-prolyl-(S)-arginyl-);
(2S,3R)-AHCA-cyclo(-$N^{\varepsilon}$-(S)-lysyl-(S)-prolyl-(S)-arginyl-);
(2S,3R)-3-amino-2-hydroxy-4-(4-hydroxyphenyl)butanoyl-cyclo(-$N^{\varepsilon}$-(S)-lysyl-(S)-prolyl-(S)-arginyl-);
(2S,3R)-3-amino-2-hydroxybutanoyl-cyclo(-$N^{\varepsilon}$-(S)-lysyl-(S)-prolyl-(S)-arginyl-);
(2S,3R)-AHPA-cyclo(-$N^{\delta}$-(S)-ornithyl-(S)-prolyl-(S)-arginyl-);
(2S,3R)-3-amino-2-hydroxy-4-(4-methoxyphenyl)butanoyl-cyclo(-$N^{\varepsilon}$-(S)-lysyl-(S)-prolyl-(S)-arginyl-);
(2S,3R,4S)-3-amino-2,4-dihydroxypentanoyl-cyclo(-$N^{\varepsilon}$-(S)-lysyl-(s)-prolyl-(s)-arginyl-);
$N^{\alpha}$-((2S,3R)-AHCA)-(S)-lysyl-(S)-prolyl-(S)-arginine;
$N^{\alpha}$-((2S,3R)-3-amino-2-hydroxy-4-(4-methoxyphenyl)butanoyl)-(S)-lysyl-(S)-prolyl-(S)-arginine;
$N^{\alpha}$-((2S,3R)-3-amino-2-hydroxybutanoyl)-(S)-lysyl-(S)-prolyl-(S)-arginine;
$N^{\alpha}$-((2S,3R)-AHCA)-(S)-leucyl-(S)-prolyl-(S)-arginine; and
$N^{\alpha}$-((2S,3R)-AHCA)-(S)-β-cyclohexylalanyl-(S)-prolyl-(S)-arginine;
and a pharmaceutically active salt thereof.

**6.** A process for the production of a compound represented by the formula (I) according to claim 1, comprising the steps of:
(1) reacting a compound represented by the following formula (IV):

$$H_2N-\overset{\overset{R^1}{|}}{C}H-\overset{\overset{X}{|}}{C}H-COOH \qquad (IV)$$

wherein $R^1$ and X have the same meanings as defined in claim 1, or a corresponding compound wherein a amino group, a functional group if present in the $R^1$, and/or a mercapto or hydroxy group if X is a mercapto or hydroxy group, are protected, with a compound represented by the following formula (V):

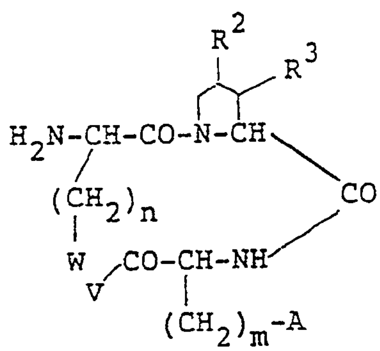

(V)

wherein V, W, $R^2$, $R^3$, A, m and n have the same meaning as defined in claim 1, or with a corresponding compound-B-(V) wherein one or more functional groups in the formula (V) are protected, in the presence of a condensation agent, and if necessary in the presence of a base; and optionally, (2) eliminating the protective groups.

7. A pharmaceutical preparation comprising a peptide derivative according to claim 1 and a pharmaceutically acceptable carrier.